# FASCICULE DE BREVET EUROPEEN

(11) **EP 0 499 501 B1**
(45) Date de publication et mention de la délivrance du brevet: **19.08.1998**
(21) Numéro de dépôt: 92400174.6
(22) Date de dépôt: 22.01.1992
(51) Int. Cl.: C07D 405/00, C07D 403/06, C07D 493/04, C07D 405/04, C07F 9/6558, A61K 51/04, A61K 51/06

(54) **Nouveaux ligands macrocycliques azotés, procédé de préparation, complexes polymétalliques, composition de diagnostic et thérapeutique**
Neue makrocyclische Stickstoff-Liganden, Verfahren zu ihrer Herstellung, Metallkomplexe, diagnostische und therapeutische Zusammensetzungen
New macrocyclic nitrogen ligands, method for their preparation, metal complexes, diagnostic and therapeutic compositions

(30) Priorité: 24.01.1991 FR 9100811
(43) Date de publication de la demande: 19.08.1992
(73) Titulaire: GUERBET S.A., F-93430 Villepinte (FR)
(72) Inventeur: Rousseaux, Olivier, F-93600 Aulnay sous Bois (FR); Schaefer, Michel, F-77400 Lagny (FR); Bouillot, Anne, F-92100 Boulogne-Billancourt (FR); Meyer, Dominique, F-94100 Saint Maur des Fosses (FR)
(74) Mandataire: Le Guen, Gérard

(56) Documents cités:
- EP-A- 0 299 795
- EP-A- 0 374 929
- TETRAHEDRON, vol. 45, no. 1, janvier 1989, pages 219-226, Pergamon Press, GB; I.M. HELPS et al.: "General routes for the synthesis of mono, d and tri-N- substituted derivatives of cyclam"
- JOURNAL OF THE CHEMICAL SOCIETY, CHEMICAL COMMUNICATIONS, no. 12, juin 1989, pages 797-798; J.P.L. COX et al.: "Synthesis of a kinetically stable yttrium-90 labelled macrocycle-antibody conjugate"

## Description

La présente invention concerne de nouveaux ligands cycliques azotés et des complexes métalliques formés par ces ligands, les applications diagnostiques et thérapeutiques de ces complexes notamment en imagerie par résonance magnétique, en radiologie par rayons X, comme agents de déplacement chimique in vivo et en médecine nucléaire.

L'invention concerne également un procédé de préparation de ces ligands.

EP-A-0299 795 décrit des dérivés d'acides aminopolycarboxyliques linéaires et cycliques, les chelates métalliques qu'ils forment et leurs applications notamment comme agents de contraste en imagerie médicale; ces dérivés comportent au moins un groupe hydrophile avec un hydroxyle sur la chaîne alkylène liant 2 atomes d'azote et un substituant portant une fonction acide carboxylique sur 2 des atomes d'azote.

Tetrahedron, Vol. 45 (1989), p. 219-226, décrit des procédés de préparation de dérivés mono, di ou tri substitués du 1, 4, 8, 11-tétraazacyclotétradécane.

Dans le Journal of the Chemical Society, Chemical Communications N° 12 (1989), p. 798-799 la vitesse et le mécanisme de coordination d'ions métalliques de transition labiles avec des macrocycles tri et tétraazotés comportant un bras latéral 2,2'-bipyridyl-6-ylméthyl, sont étudiés.

Plus précisément, l'invention a pour objet des ligands de formule II 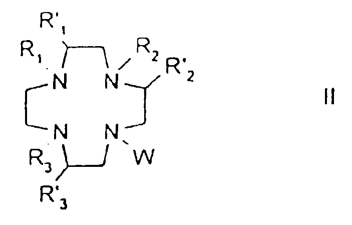 dans laquelle
R₁, R₂, R₃ identiques ou différents sont choisis parmi un atome d'hydrogène, et les groupes 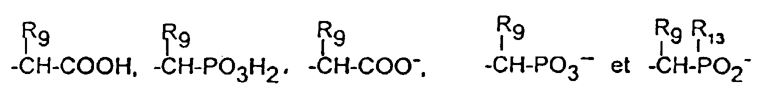
Rg représentant un atome d'hydrogène, un groupe alkyle à chaîne linéaire ou ramifiée en C₁-C₆, hydroxyalkyle à chaîne linéaire ou ramifiée en C₁-C₆, polyhydroxyalkyle à chaîne linéaire ou ramifiée en C₁-C₆, alkoxy (en C₁-C₆) alkyle en C₁-C₆ à chaîne linéaire ou ramifiée, R₁₃ représentant un groupe alkyle à chaîne linéaire ou ramifiée en C₁-C₆, un groupe hydroxy- ou polyhydroxyalkyle à chaîne linéaire ou ramifiée en C₁-C₆, un groupe alkoxy (en C₁-C₆) alkyle à chaîne linéaire ou ramifiée en C₁-C₆, un groupe hydroxy- ou polyhydroxyalkoxy (en C₁-C₆) alkyle en chaîne linéaire ou ramifiée en C₁-C₆, sous réserve qu'au moins deux groupes parmi R₁, R₂ ou R₃ représentent 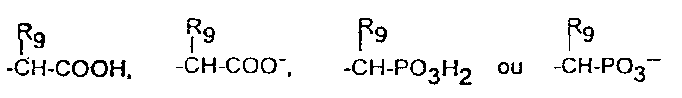
R'₁, R'₂ ou R'₃, identiques ou différents représentant un atome d'hydrogène, un groupe alkyle à chaîne linéaire ou ramifiée en C₁-C₆, un groupe hydroxyalkyle ou polyhydroxyalkyle à chaîne linéaire ou ramifiée en C₁-C₆, W est choisi parmi les groupes suivants : 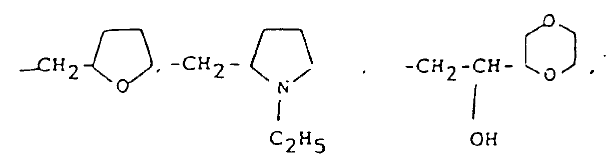 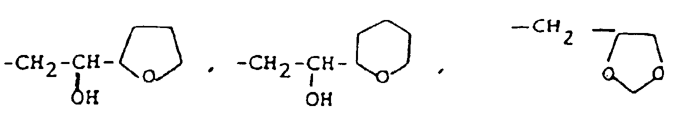 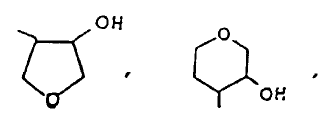 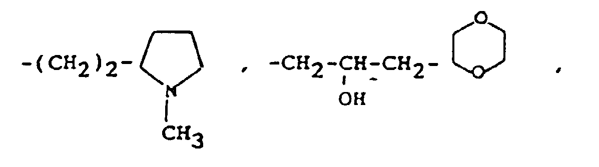 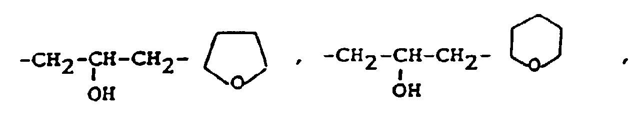 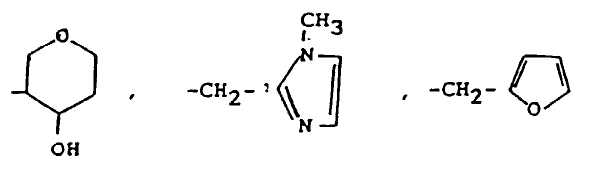 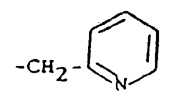
ou W représente un groupe 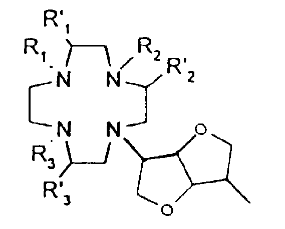 dans lequel R₁, R₂, R₃, R'₁, R'₂ et R'₃ sont tels que définis précédemment, ainsi que les sels de ces composés formés avec des bases minérales ou organiques, ou des aminoacides basiques.

On préfère en particulier les composés de formule générale Il dans laquelle R₁, R₂ et R₃ représentent le groupe CH₂-COOH.

Parmi ces composés, on préfère en outre le groupe de composés de formule générale III: 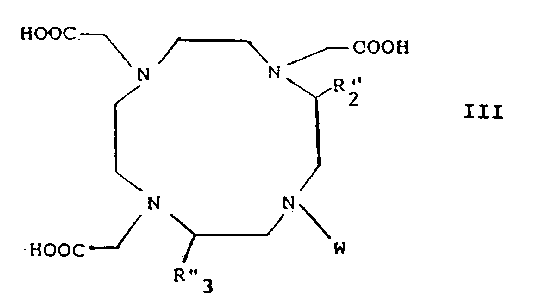 dans laquelle R"₂ et R"₃ sont choisis parmi un atome d'hydrogène, le groupe méthyle et le groupe éthyle, W étant tel que défini précédemment.

Parmi les composés de formule III que l'on préfère, l'on peut citer les composés dans lesquels R"₂ et R"₃ représentent un atome d'hydrogène et W représente les groupes :

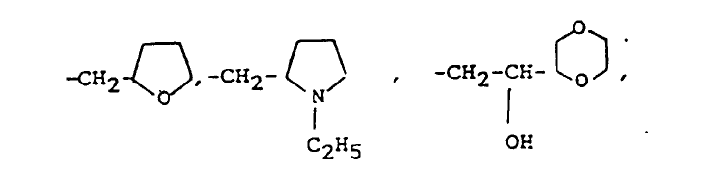

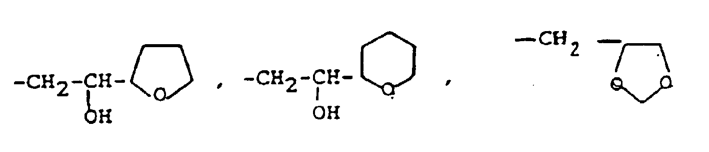

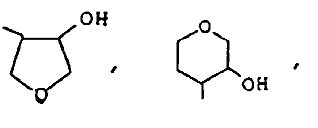

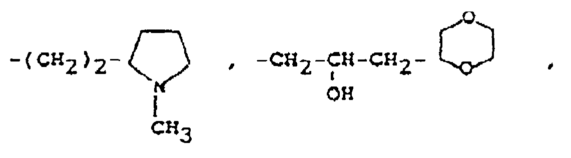

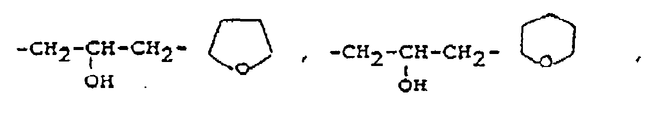

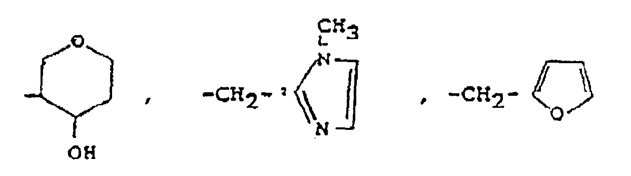

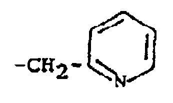

Sont également préférés les composés de formule III dans lesquels W représente : 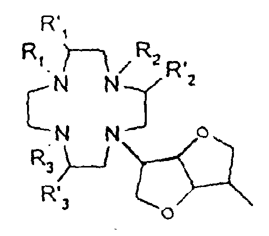 et R"₂, R"₃ sont choisis parmi un atome d'hydrogène, le groupe méthyle et le groupe éthyle.

Un autre groupe de composés préférés est constitué des composés de formule III 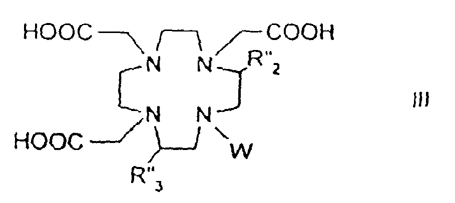 dans laquelle R"₂ et R"₃ représentent le groupe méthyle et W représente :

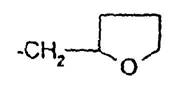

On préfère en particulier les composés de formule II suivants :

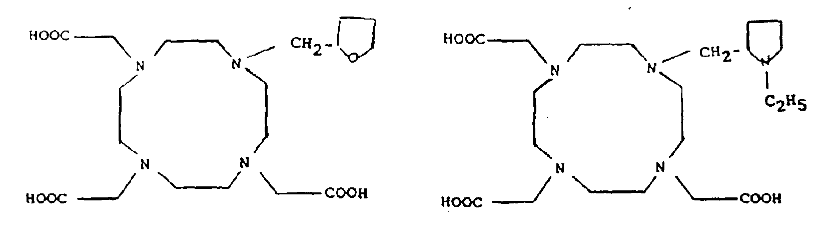

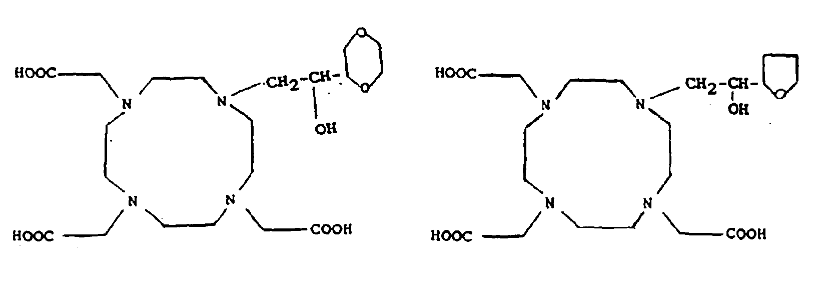

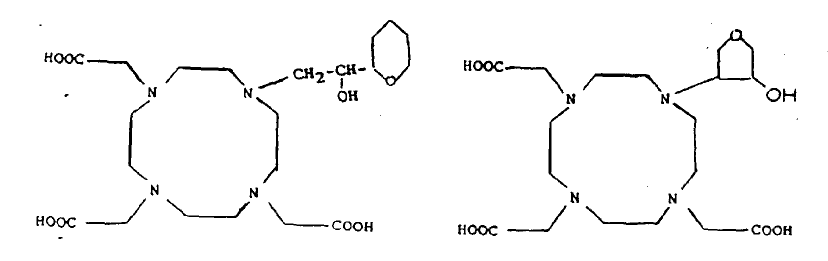

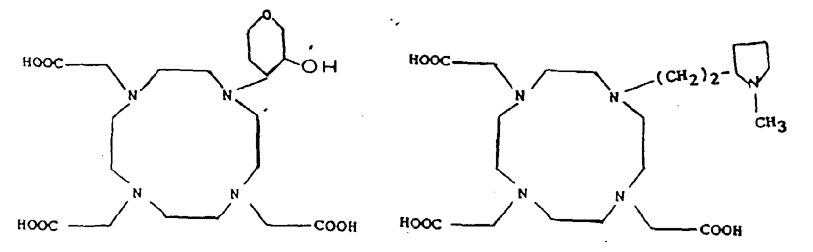

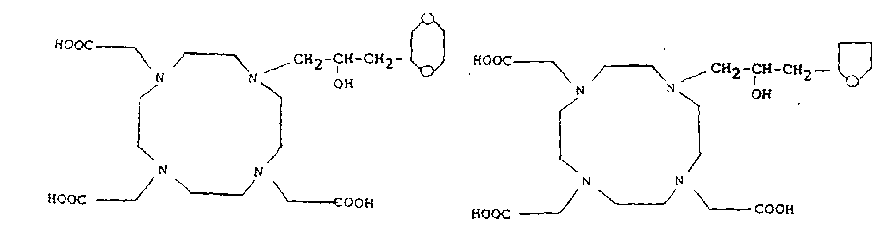

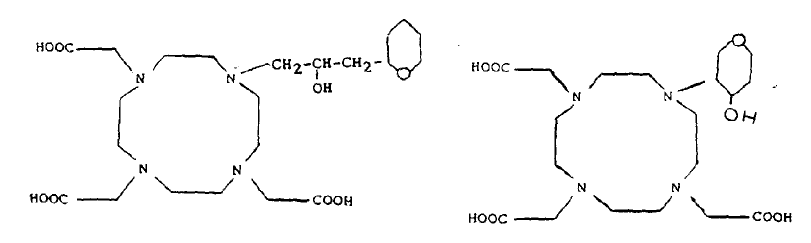

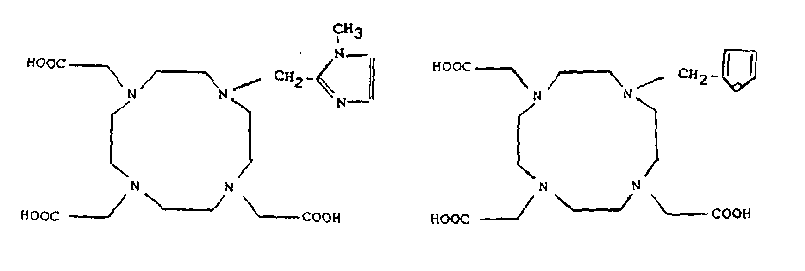

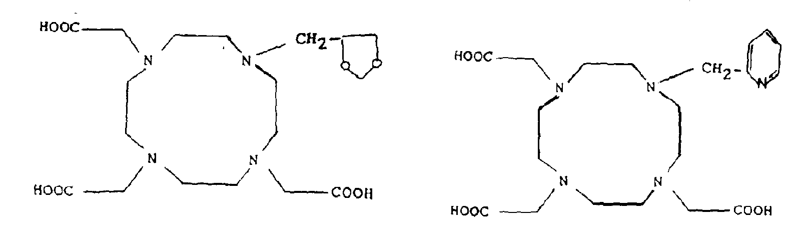

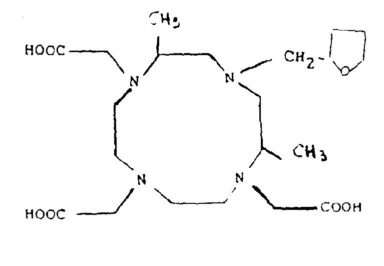

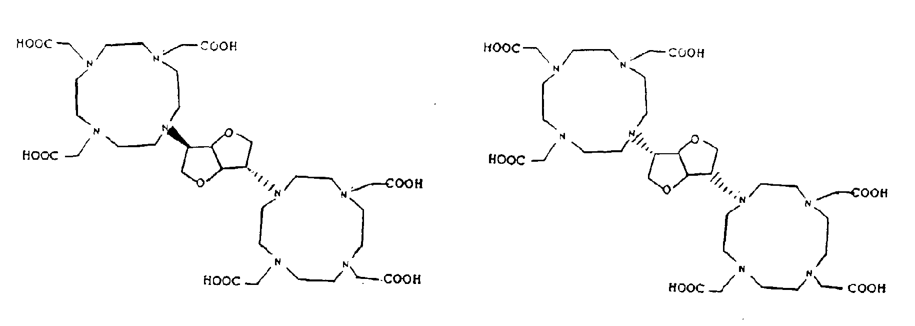

Les ligands de formule II peuvent être préparés par la succession d'étapes suivantes :
a) réaction d'un composé de formule 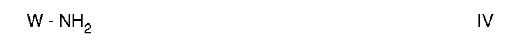 dans laquelle W est tel que défini précédemment avec un composé de formule 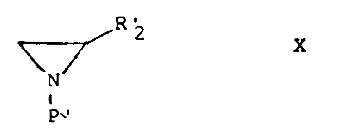 dans lequel R'₂ est tel que défini précédemment et P' représente un groupe tosyle, mésyle, phénylsulfonyle, ou 4-méthoxyphénylsulfonyle, pour obtenir un composé de formule XI 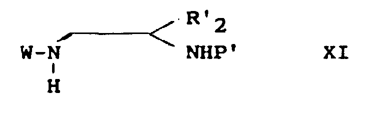
b) réaction du composé de formule XI avec un composé de formule XII 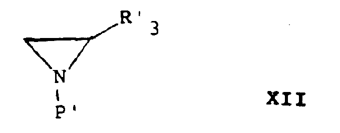 R'₃ et P' étant tels que définis précédemment pour obtenir le composé de formule XIII 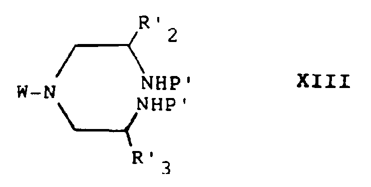
c) réaction du composé de formule XIII avec un composé de formule 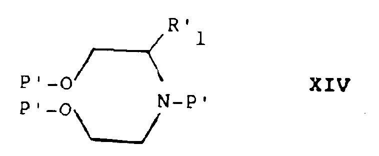 P' et R'₁ étant tels que définis précédemment pour obtenir un composé de formule XV 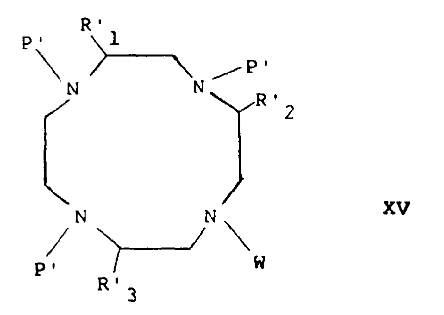
d) élimination du groupe P' pour obtenir le composé de formule XVI, 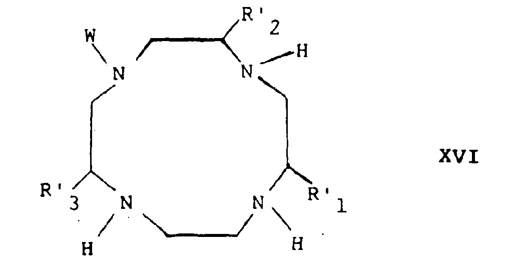
e) alkylation du composé de formule XVI.

Les réactions des étapes a) et b) sont effectuées à une température de l'ordre de 40 à 100°C dans un solvant tel que le DMF, le toluène ou l'acétonitrile.

La réaction de l'étape c) a lieu dans le DMF en présence de NaH à chaud comme décrit dans Org. Synth. 1979, vol. 58, p. 86-97 ou avec le carbonate de césium dans du DMF sec à une température de l'ordre de 40 à 80°C comme décrit dans J. Org. Chem. 1984, vol. 49, p. 110-113 ou encore par catalyse par transfert de phase.

L'élimination du groupe tosyle peut être réalisée notamment par réaction des composés de formule IX,
soit avec l'acide bromhydrique dans l'acide acétique au reflux, comme décrit dans WO-8 602 352
soit avec l'acide sulfurique concentré à environ 100°C comme décrit dans EP-287 465
soit avec du sodium dans l'ammoniac liquide comme décrit dans Helvetica Chimica Acta, vol. 71, 1988, p. 685 soit avec du sodium dans le butanol à une température d'environ 100°C comme décrit dans Helvetica Chimica Acta, vol. 73, 1990, p. 716.

La réaction d'alkylation peut être réalisée:
soit de manière directe par réaction du composé obtenu à l'étape c) avec de l'acide chloracétique ou un chloracétate comme décrit dans EP-287 465
soit de manière indirecte par réaction du composé obtenu à l'étape c) avec un ester de l'acide α-bromo-acétique ou a-chloroacétique tel que le bromacétate de tert-butyle ou le chloracétate de tert-butyle dans le DMAC ou le DMF en présence d'une base comme décrit dans EP-299 795 ou le bromacétate d'éthyle dans l'éthanol en présence de carbonate de césium comme décrit dans J. Chem. Soc. Chem. Commun. 1989, p. 794, suivie d'une saponification du composé obtenu par une méthode connue de saponification ou par l'acide trifluoroacétique en excès à température ambiante dans le cas des esters de tertiobutyle comme décrit dans EP-299 795.

Les composés de formule XV peuvent également être préparés de la manière suivante :
a) réaction de la benzylamine avec un composé de formule X telle que décrite précédemment pour obtenir un composé de formule XVII: 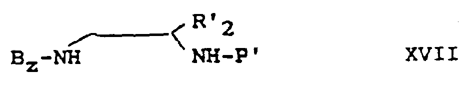 B_{z} représentant le groupe benzyle,
b) réaction du composé de formule XVII avec un composé de formule XII tel que décrit précédemment pour obtenir un composé de formule XVIII 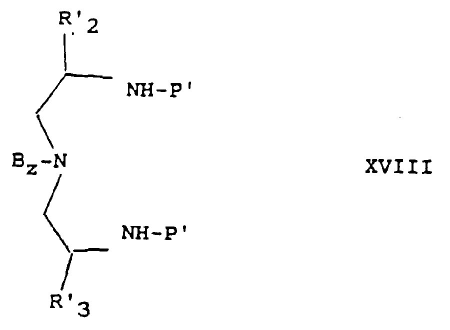
c) réaction du composé de formule XVIII avec un composé de formule XIV pour obtenir un composé de formule XIX, 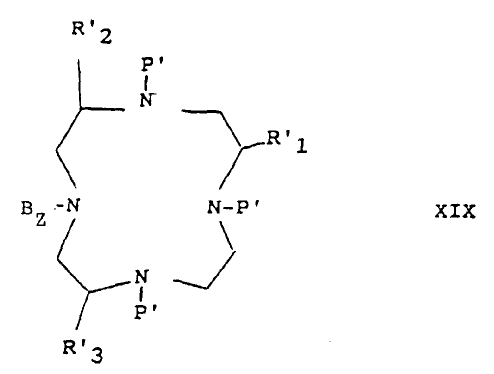
d) hydrogénation du composé XIX pour obtenir un composé de formule XX, 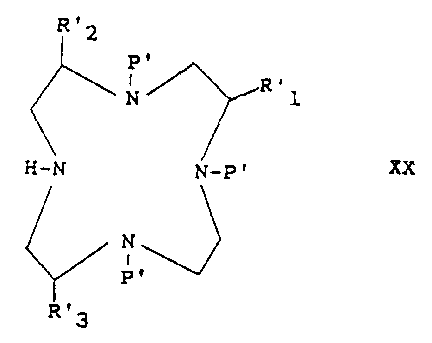
e) réaction du composé de formule XX avec un composé de formule W-P", W étant tel que défini précédemment et P" représentant le groupe P'ou un atome de chlore ou de brome pour obtenir un composé de formule XV telle que définie précédemment.

Les réactions d'élimination du groupe P' et d'alkylation ont lieu comme décrit précédemment.

Les amines de formule IV sont des produits connus, comme par exemple la 2-aminométhyl-1-éthyl pyrrolidine, la 2-aminoéthyl-1-méthyl pyrrolidine, le 2-aminométhylfuranne, le 2-aminométhyl tétrahydrofuranne et la 2-aminométhyl pyridine commercialisés par la firme ALDRICH, ou peuvent être préparés par exemple de la manière suivante :
a) époxydation d'un composé de formule XXI : 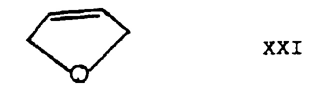 par de l'acide chloroperbenzoique, comme décrit dans Journal of Pharmaceutic Sciences, 1970, vol. 59, p. 1676-1679,
b) réaction du composé obtenu avec un azoture tel que NaN₃ dans du méthanol aqueux en présence de chlorure d'ammonium tel que décrit dans Tetrahedron Letters vol. 31, 1990, p. 5641-5644, pour obtenir un composé de formule XXII 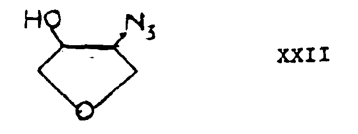
c) réduction du composé de formule XXII par de l'hydrogène sur charbon palladié comme décrit dans synthes is 4, 1990, p. 366-368, pour obtenir l'amine correspondante de formule XXIII

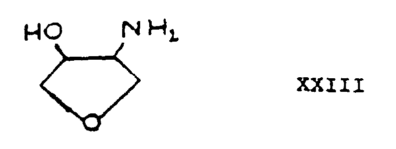

La même procédure peut être répétée pour préparer les amines suivantes : 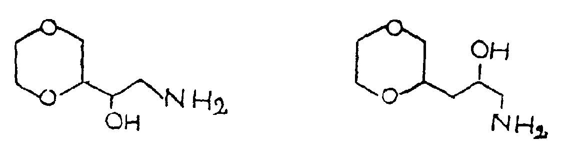 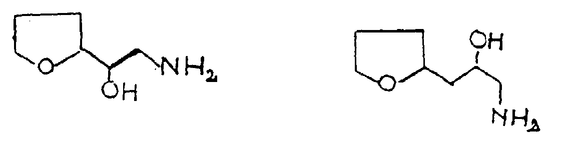 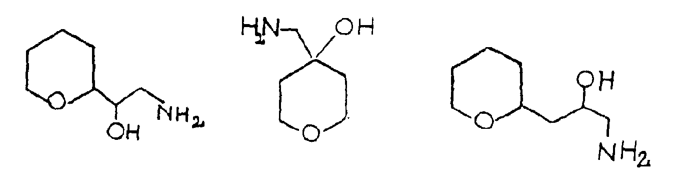 à partir des composés suivants :

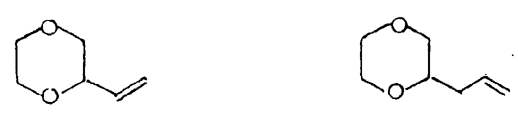

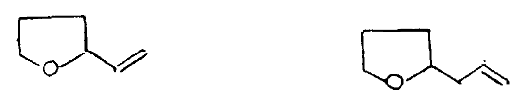

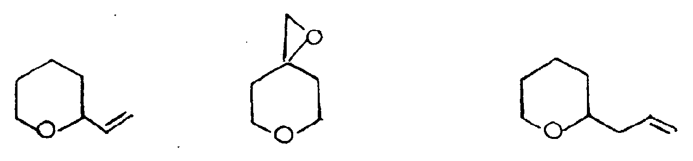

Les procédés de préparation de ces composés sont décrits de manière plus détaillée par Normant, Castro, C.R. Acad. Sciences, 259, 1964, p. 830; Ficini, Bull Soc. Chim. Fr., 1956, p. 119-123; Mikailovic, Helv. Chim. Acta, vol. 56, n° 8, 1973, p. 3056; Colonge, Buendia, C.R. Acad. Sciences, 261, 1965; Olsen, Chem. Ber. vol. 91, 1958; p. 1589-1594.

L'amine de formule 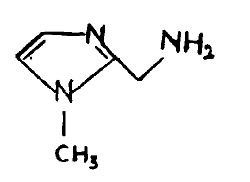 peut en outre être préparée par réaction du 3-méthylimidazole avec du formaldéhyde, pour obtenir le composé de formule 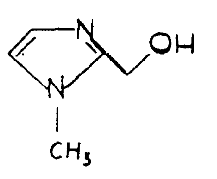 que l'on met à réagir avec SOCl₂ pour obtenir 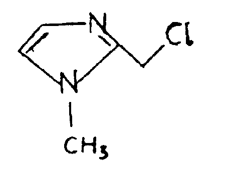 suivie d'une réaction du produit obtenu avec NaN₃ suivie d'une hydrogénation catalytique.

Les composés de formules X et XII peuvent être préparés par réaction d'un composé de formule 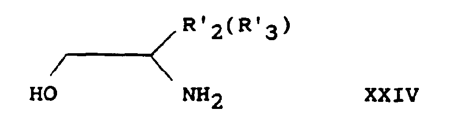 R'₂ et R'₃ étant tels que définis précédemment avec un composé de formule P'-Cl, P' étant tel que défini précédemment pour obtenir le composé de formule 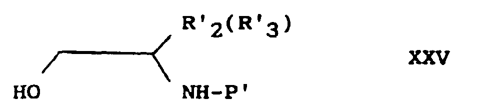 que l'on met à réagir avec KOH aqueux pour obtenir le composé deformule X ou XII correspondant, comme décrit dans Helvetica Chimica Acta, vol. 68, 1985, p. 289, ou en faisant réagir un composé de formule 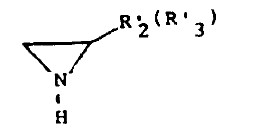 avec le chlorure de tosyle en présence de pyridine de façon à obtenir un composé de formule

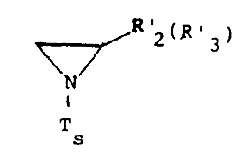

La présente invention a en outre pour objet des complexes formés par au moins un ligand de formule II avec au moins un ion métallique choisi parmi les ions des lanthanides, les ions des métaux de transition, le baryum, le bismuth, le plomb et les radioisotopes ^{99m}Tc, ¹¹¹ln, ⁹°Y, ⁶⁴Cu et ⁷⁶⁹Yb, ainsi que les sels de ces complexes avec des bases minérales ou organiques pharmaceutiquement acceptables ou des aminoacides basiques sont particulièrement intéressants.

Les complexes sont mono- ou polymétalliques, de préférence mono- ou bi-métalliques, neutres ou ioniques.

Les complexes mono- ou bimétalliques dans lesquels l'ion métallique est choisi parmi le gadolinium, l'europium, le dysprosium, le fer (Fe³+), le manganèse (Mn²+) et le baryum sont particulièrement préférés.

Comme exemples de sels, on peut citer ceux formés avec l'hydroxyde de sodium, la N-méthylglucamine, la diéthanolamine, la lysine et l'arginine.

Les complexés peuvent être obtenus par réaction des ligands avec un sel ou un oxyde des métaux dans un solvant aqueux et éventuellement neutralisation pour former un sel.

Il va de soi que la présente invention englobe non seulement les ligands de formule Il et les complexes précédemment définis sous forme de mélange racémique mais également des stéréoisomères de ces ligands et complexes.

Les complexes selon l'invention formés par les ligands de formule Il peuvent être utilisés dans des applications diagnostiques in vitro et in vivo chez l'homme et chez l'animal.

En imagerie par résonance magnétique et en spectroscopie RMN in vivo, ils peuvent être utilisés comme agents de relaxation; à cet effet on préfère les complexes formés avec les métaux suivants : Gd³⁺, Mn²⁺ et Fe³⁺; comme agents de susceptibilité magnétique : à cet effet, on préfère les complexes formés avec les métaux Dy³+, Ho³+,Tb³+ et Er3+; ou comme agents de déplacement chimique : à cet effet on préfère les complexes formés avec les métaux Eu3+, Pr³+ et Yb³+.

Les complexes formés par les ligands de formule II et les métaux choisis de préférence parmi les lanthanides, La3+, Bi²⁺, Ba2+ et Pb²⁺ peuvent être utilisés en imagerie par rayons X.

En médecine nucléaire, les complexes formés par les ligands de formule Il et les métaux ^{99m}Tc, ¹¹¹In, ⁶⁴Cu et ¹⁶⁹Yb, peuvent être utilisés dans des applications radiodiagnostiques et les complexes formés avec les métaux ⁹⁰Y, 111 In, ¹⁶⁹Yb, ²¹²Bi et ⁶⁴C_{U} peuvent être utilisés dans des applications radiothérapeutiques après couplage du complexe à une biomolécule appropriée.

Les complexes formés par les ligands de formule Il et les ions métalliques choisis de préférence parmi Eu3+ et Tb³+ peuvent être utilisés dans des applications diagnostiques in vitro par photoluminescence, telles que des fluoroim- muno-essais.

La présente invention a en conséquence également pour objet une composition de diagnostic administrable à l'homme, caractérisée en ce qu'elle comprend au moins un complexe formé par un ligand de formule I avec au moins un ion métallique choisi parmi les ions des lanthanides, les ions des métaux de transition, du baryum, du bismuth, du plomb et des _{rad}iₒiₛₒtₒpₑₛ suivants 99m_{Tc}, ¹¹¹In, ⁹⁰y, ⁶⁴Cu et ¹⁶⁹Yb, ainsi que les sels de ces complexes avec des bases minérales ou organiques pharmaceutiquement acceptables ou des aminoacides basiques.

Ces compositions peuvent être constituées notamment par des solutions dans un solvant aqueux physiologiquement acceptable d'un complexe selon l'invention.

Avantageusement, une formulation des compositions de l'invention comprend en plus d'au moins un complexe selon l'invention, de 0,05 à 10% en mole du ligand correspondant sous forme de complexe calcique ou de zinc.

Les compositions de diagnostic selon l'invention peuvent être administrées :
par voie parentérale dont la voie intraveineuse, la voie infra-artérielle, la voie intra-lymphatique; la voie sous cutanée
par voie orale
par voie sous-arachnoidienne
par voie intrabronchique sous forme d'aérosol.

En imagerie par résonance magnétique, les doses sont très variables selon les voies d'administration.

Pour la voie intraveineuse ou intra-artérielle, la dose est d'environ 0,01 à 2 mM/kg.

Pour la voie orale cette dose peut aller jusqu'à 10 mM/kg.

Pour les autres voies d'administration, les doses utiles sont généralement inférieures à 1 mM/kg et même généralement pour la voie sous-arachnoidienne inférieures à 0,05 mM/kg.

Dans l'utilisation comme agents de déplacement chimique pour la spectroscopie in vivo, comme agents de susceptibilité magnétique en IRM et comme agents de contraste en radiologie par rayons X les doses sont les mêmes, sauf par voie intraveineuse ou intra-artérielle où les doses peuvent être de 0,2 à 5 mM/kg.

Les exemples suivants illustrent la préparation de composés selon la présente demande.

### EXEMPLE 1 :

Préparation du composé de formule :

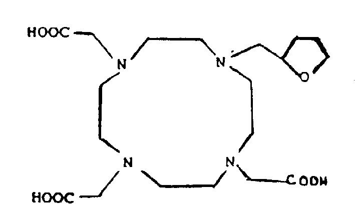

### a) préparation du composé de formule :

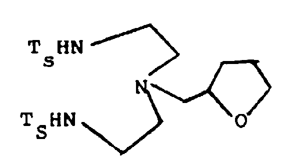

8,3g (82 mmoles) de tétrahydrofurfurylamine et 32,4 g (164 mmoles) de tosylaziridine sont dissous dans 500 cm³ de toluène sec à une température de 100°C. Le mélange est agité pendant 20h à cette température. Le milieu réactionnel est ensuite filtré à chaud et le solvant est évaporé. L'huile obtenue est filtrée sur un bouchon de silice. Après évaporation du solvant, on obtient 28,4 g d'un produit gommeux qui cristallise lentement.

Rendement : 70%.

CCM : silice; CH₂Cl₂/acétate d'éthyle : 90/10; Rf: 0,3.

### b) Préparation du composé de formule :

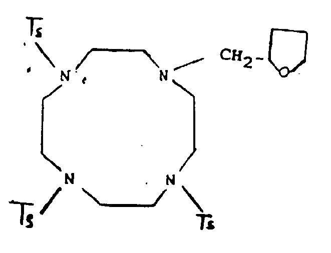

9,9g (20 mmoles) de produit préparé en a) sont dissous dans 1200 cm³ de DMF sec. Le réacteur étant placé sous atmosphère d'argon, on ajoute 30 g de Cs₂CO₃ pulvérulent sec et on porte le mélange à 30°C. Une solution de 11,35 g de N-tosyl-bis(2-tosyloxy éthyl)amine dans 300 cm³ de DMF est introduite goutte à goutte. Le mélange réactionnel est agité jusqu'à disparition des produits de départ. Lorsque la réaction est terminée, le mélange est filtré et le solvant est évaporé sous pression réduite. Le résidu est repris avec 500 cm³ de dichlorométhane. La solution chlorométhylénique est filtrée et lavée trois fois à l'eau. Après séchage sur MgS0₄, le dichlorométhane est évaporé. Le résidu est purifié par chromatographie sur silice. On obtient 7 g de produit pur (Rendement : 49%).

CCM : silice; CH₂Cl₂/acétate d'éthyle :95/5; Rf : 0,4.

RMN ¹³C : 150-130 ppm, groupes aromatiques, groupes tosyle; 80, 71,8, 53, 34,8 et 29,8 ppm, motif tétrahy- drofurfuryle; 61-53 ppm, macrocycle; 26, 24 ppm CH₃ des groupes tosyle.

### c) Préparation du composé de formule :

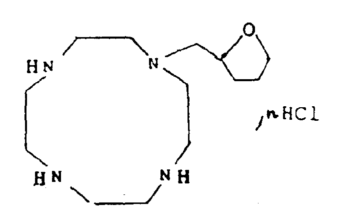

A 250 cm³ de n-butanol sec, on ajoute 5g de produit obtenu en b). Le mélange est agité sous atmosphère d'azote sec et porté à la température de 100°C. Après dissolution complète, on ajoute par petites portions 15 g de sodium à la solution. Lorsque l'addition est terminée, le butanol est évaporé et le résidu est repris à l'eau. La solution aqueuse est évaporée et le résidu est repris à l'éthanol absolu.

Après évaporation de l'éthanol, on reprend le produit au dichlorométhane et on filtre les sels. La solution chlorométhylénique est évaporée et le résidu huileux obtenu est dissous dans 200 cm³ d'acide chlorhydrique 1 M. La solution acide est évaporée à sec. Après séchage on obtient 1,8 g de produit sous forme de chlorhydrate.

CCM : silice-dioxanne/eau/ammoniaque : 8/3/2; Rf : 0,1.

### d) Préparation du composé de formule :

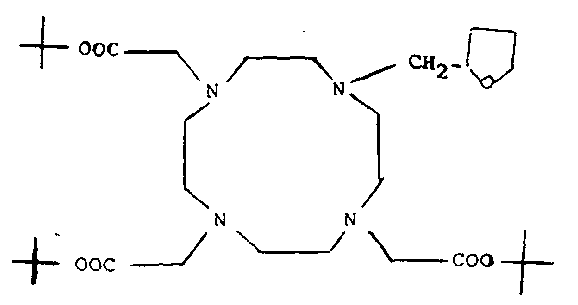

580 mg de produit préparé en c) et 1,5 g d'hydrogénocarbonate de sodium sont mis en suspension dans 40 cm³ de DMF sec. Le réacteur est placé sous atmosphère d'argon et le mélange porté à une température de 50°C. On ajoute alors goutte à goutte une solution de 1 cm³ de bromacétate de tertiobutyle et 10 cm³ de DMF sec. Après 24h, le DMF est évaporé sous pression réduite. Le produit est alors dissous dans CH₂Cl₂ et séparé des sels par filtration. Après évaporation du dichlorométhane, on obtient une huile jaune qui est purifiée par chromatographie sur silice. On obtient 650 mg de produit pur qui cristallise lentement.

CCM : silice, CH₂Cl₂/méthanol 10%; Rf : 0,5.

I.R. : 1710 cm⁻¹ bande du carbonyle.

RMN ⁺H: 1,43 ppm, singulet, tertiobutyle.

RMN ¹³C : 173 ppm, carbonyle; 83 ppm, C quaternaire terbutyle; 28 ppm, groupes méthyle; 74, 69, 30, 26 ppm motif tétrahydrofuranne.

Spectre de masse : MH⁺ 599.

### e) Préparation du composé de formule :

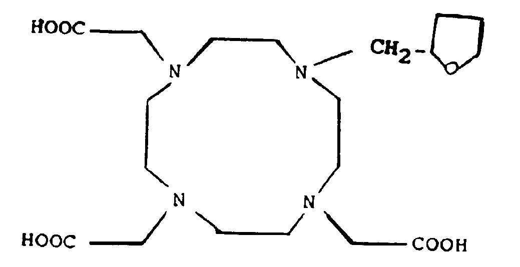

650 mg de produit préparé end) sont dissous dans 10 cm³ d'acide trifluoroacétique. Le mélange est agité pendant 24h à température ambiante. L'acide trifluoroacétique est ensuite évaporé et le résidu est coévaporé avec de l'eau.

Le produit est ensuite purifié sur une résine IRA 458 OH-. On obtient finalement 150 mg de produit.

CCM : silice; acétate d'éthyle/isopropanol/ammoniaque :12/35/30; Rf : 0,6.

RMN ¹³C : 170 ppm, carbonyle;74,65, 27, 22 ppm, motif tétrahydrofuranne.

Spectre de masse : MH⁺ 431

On prépare le complexe de gadolinium du composé décrit ci-dessus en mettant en suspension dans 100 ml d'H₂0 à 80° C Gd₂O₃ (4 mmoles) et le composé brut obtenu à l'exemple précédent (8 mmoles) et en agitant la suspension pendant 24 heures.

La solution est refroidie à température ambiante puis le solvant est chassé sous pression réduite.

Le solide résultant est dissous dans 15 ml d'H₂0 et 5 ml de C₂H₅OH et est ajouté goutte à goutte à 750 ml d'acétone placée sous agitation rapide.

Le précipité qui se forme est filtré puis lavé avec de l'acétone et séché sous vide à température ambiante.

### EXEMPLE 2:

Préparation du composé de formule :

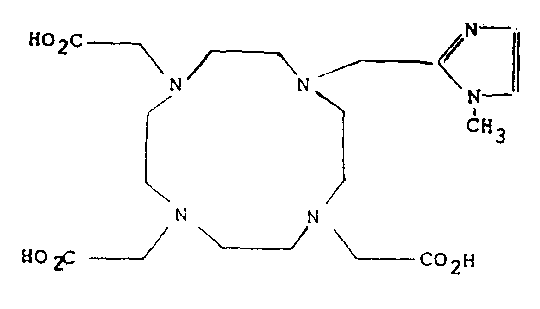

### a) Préparation du composé de formule :

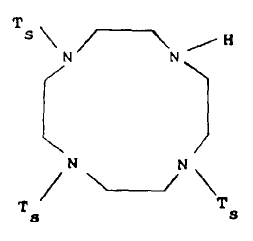

29 g (40 mmoles) de monobenzyl tritosylcyclène préparés selon le procédé décrit dans EP-255 471 sont réduits sous une pression de 4.10⁵ Pa d'hydrogène en présence de charbon palladié dans 500 cm³ d'acide acétique. Le mélange est agité pendant 24 h à 70°C. Après filtration, le solvant est évaporé. Le solide obtenu est lavé successivement avec une solution de carbonate de potassium puis à l'eau. 23,7 g de poudre blanche sont obtenus.
CCM : Si0₂; CH₂Cl₂/CH₃OH (95/5) Rf 0,3; PF = 195°C

Rendement : 93%.

### b) Préparation du composé de formule

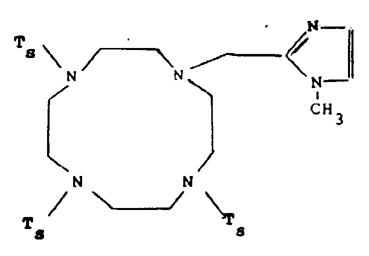

5 g (7,8 mmoles) de tritosylcyclène préparés en a), 1,45 g (8,6 mmoles) de 2-chlorométhyl 1-méthyl imidazole tel que décrit dans JOCELYN P.C., J. Chem. Soc., 3305, (1957) et 2,7 cm³ (19,7 mmoles) de triéthylamine sont dissous dans 50 cm³ de dichlorométhane. Le milieu réactionnel est chauffé au reflux pendant 1 h. On ajoute 1,45 g (8,6 mmoles) de réactif chloré à la solution. On chauffe au reflux pendant 12 h On ajoute 1,35 cm³ (9,85 mmoles) de triéthylamine en solution dans 10 cm³ de dichlorométhane et le milieu réactionnel est chauffé de nouveau pendant 12h au reflux. Après refroidissement, la triéthylamine est éliminée par lavage à l'eau. La phase organique séchée sur MgS0₄ est concentrée puis purifiée par chromatographie sur silice. 6 g de poudre blanche sont obtenus.
CCM : Si0₂; CH₂Cl₂/CH₃OH (90/10) Rf = 0,6,
Rendement : 53%.
RMN H (DMSO) δ = 2,41 ppm : 2s, CH₃
δ = 2,65 ppm : m, CH₂
3,05 ppm < δ < 3,7 ppm : m, CH₂
6,7 ppm < 8 < 7,05 ppm : d, CH = CH
7,4 ppm < δ < 7,75 ppm : m, CH = CH

### c) Préparation du composé de formule :

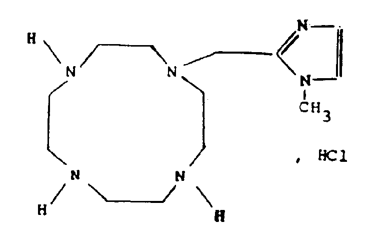

6 g (8,3 mmoles)de substrat préparé en b) sont dissous dans 80 cm³ de n-BuOH sec. La solution placée sous atmosphère inerte est chauffée à 100°C. 5 g (220 mmoles) de sodium sont ajoutés très lentement à la solution. Celle-ci est ensuite maintenue pendant 12 h à 100° C. Le solvant est évaporé. Le produit est repris plusieurs fois à l'eau et séché avant d'être extrait par le dichlorométhane (trois fois 20 cm³). Le solvant est évaporé et le résidu dissous dans 50 cm³ d'acide chlorhydrique 6N. La solution est concentrée et lavée trois fois avec 15 cm³ de dichlorométhane. Après séchage, 2,9 g de produit sont isolés sous forme de chlorhydrate.

CCM : Si0₂; CHCl₃/CH₃OH/NH₄OH (4/4/2). Rf = 0,3

### d) Préparation du composé de formule :

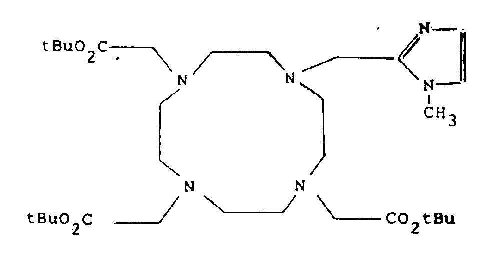

2,2 g (5,8 mmoles) de produit préparé en c) et 6,3 g (58 mmoles) de carbonate de sodium sont mis en suspension dans 35 cm³ de DMF sec. Le mélange est placé sous atmosphère inerte et chauffé à 60°C. 3,75 g (19,2 mmoles) de bromoacétate de terbutyle en solution dans 35 cm³ de DMF sont ajoutés goutte à goutte Après 24 h de réaction, le DMF est évaporé. Le produit est repris dans 100 cm³ de dichlorométhane et lavé trois fois avec 70 cm³ d'eau. La phase organique est séchée sur MgSO_{4;} concentrée et purifiée par chromatographie sur silice. Après évaporation du solvant et séchage, on isole 1;4 g de poudre blanche. Rendement 40%.
CCM : Si0₂; CH₂Cl₂/EtOH (90/10); Rf= 0,5.
IR (KBr) : 1720 cm-¹ C = 0.
RMN H+(DMSO): δ = 1,4 ppm : 2s, C(CH₃)₃ 2<δ<3,5ppm : m; CH₂ = CH 6,5 < δ < 7,05 ppm : d, CH = CH
RMN¹³C (DMSO) δ= 27,4 ppm, CH₃ δ = 31,7 ppm, CH₃ δ = 48,9 ppm, CH₂ δ = 55,0 ppm, CH₂ δ = 55,7 ppm, CH₂ δ = 80,9 ppm, C δ = 121,0 ppm, HC = CH δ = 125,5 ppm, HC = CH δ = 146,0 ppm, C = N δ = 171,6 ppm, C = O δ = ₁₇2,3 ppm, C = O

spectre de masse (FAB) M/Z = 632 (M + Na+)

### e) Préparation du composé de formule :

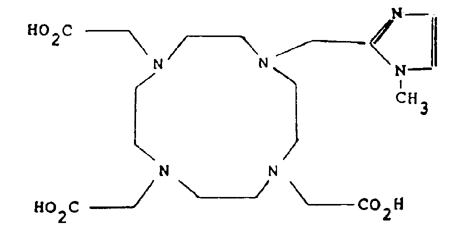

1 g (1,64 mmole) de produit préparé en d) est dissous dans 40 cm³ d'acide trifluoroacétique. La solution placée sous atmosphère inerte est agitée pendant 20 h à température ambiante. L'acide trifluoroacétique est ensuite évaporé puis azéotropé avec de l'eau. Le produit brut est purifié sur une résine IRA 458. 600 mg de poudre blanche sont isolés. Rendement 83%.

CCM : SiO₂; AcOEt/Isopropanol/NH₄OH; Rf = 0,5.
IR (KBr) 3450 cm-1 OH 1650 cm⁻¹ C = O
RMN H⁺ (DMSO) 2,6 < δ < 4 ppm : m; CH₂ et CH₃ 6,8 < δ < 7,1 ppm : d; CH = CH δ = 7,4 ppm : m; OH
RMN ¹³C (DMSO) δ = 32,7 ppm, CH₃ 8 = 49,6 ppm, CH₂ δ = 50,7 ppm, CH₂ δ = 51,2 ppm, CH₂ δ = 54,8 ppm, CH₂ 8 = ₁22,0 ppm, CH = CH 8 = 125,7 ppm, CH = CH δ = 144,0 ppm, C = N δ = ₁₇0,₁ ppm, 2C = O δ = 171,1 ppm, 1C=O

### EXEMPLE 3:

Préparation du composé de formule :

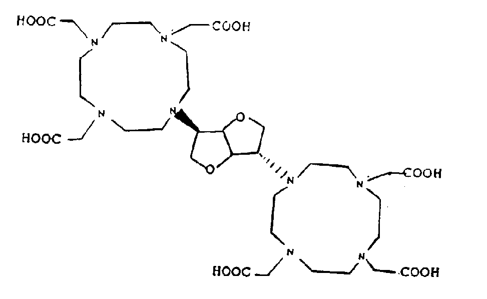

### a) Préparation du 2:5-ditosyl-1;4:3,6-dianhydrosorbitol de formule :

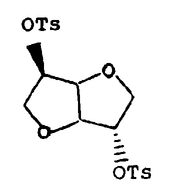

Le 2:5 ditosyl-1,4:3,6-dianhydrosorbitol est préparé à partir du 1,4:3,6-dianhydro-D-sorbitol; disponible commercialement sous le nom Isosorbide ® par la société Aldrich Chimie (Strasbourg) selon la méthode décrite dans J. Am. Chem. Soc. 68 (1946) p. 927 et J. Chem. Soc. (1946) p. 393.

Masse obtenue pour 100 g d'Isosorbide ® 213 g; rendement : 69 %. CCM : Si0₂, éluant CH₂Cl₂/ AcOEt : 97/3, Rf : 0,6.

### b) Préparation du 2:5-diazido-1,4:3,6-dianhydrosorbitol.

Le 2:5-diazido-1,4:3,6-dianhydrosorbitol est préparé à partir du composé obtenu à l'étape précédente selon la méthode décrite dans Carbohydrate Research 85 (1980) p. 259.

Masse obtenue pour 150 g du composé de l'étape précédente : 37 g; rendement 57 %; CCM : Si0₂; éluant : éther de pétrole/éther éthylique : 85/15;

Rf : 0,25.

I. R. : 2100 cm-¹ ; bande azide.

### c) Préparation du 2:5-diamino-1,4:3,6-dianhydrosorbitol.

4,5 g (22,95 mmoles) du composé obtenu à l'étape précédente sont dissous dans 200 cm³ d'éthanol à 95° C. 1 g de palladium sur charbon est ajouté à la solution puis la suspension est placée en autoclave sous une pression de 13-14.10⁵ Pa d'hydrogène, à une température de 50° C. Le milieu réactionnel est agité pendant 7 heures sous pression d'hydrogène constante puis est laissé une nuit au repos sous pression résiduelle d'hydrogène.

Le milieu réactionnel est filtré et le solvant évaporé. Le résidu huileux obtenu est repris dans l'acétonitrile et le précipité blanc formé est éliminé par filtration.

La solution obtenue est évaporée pour donner 3,3 g d'une huile incolore.

Rendement: 100 %.

I.R. : disparition de la bande azide à 2100

cm⁻¹ Bandes NH à 3360 et 3280 cm⁻¹.

RMN¹³C : 55,67 ppm - 59,17 ppm - 72,41 ppm - 75,58 ppm - 82,33 ppm - 92,68 ppm.

CCM: SiO₂; éluant CH₂Cl₂/MeOH/NH₃: 16/4/0,1.

Rf = 0,37.

### d) Préparation du composé de formule:

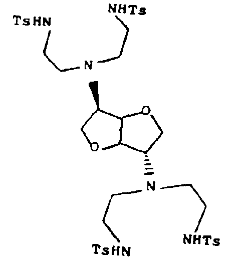

Dans un ballon tricol de 500 cm³ sont introduits 5g (34,7 mmoles) du composé obtenu à l'étape précédente et 27,3 g (0,139 mole) de tosylaziridine en solution dans 250 cm³ d'acétonitrile. Le mélange est agité et porté à 70° C pendant 4 jours. Le solvant est évaporé sous pression réduite et le résidu purifié sur colonne de silice.

17 g du produit attendu sont obtenus avec un rendement de 52 %.

CCM : SiO₂; CH₂Cl₂/MeOH : 95/5, Rf = 0,35.

I. R. : 3250 cm⁻¹ ; H-N-Ts.

### e) Préparation du composé de formule :

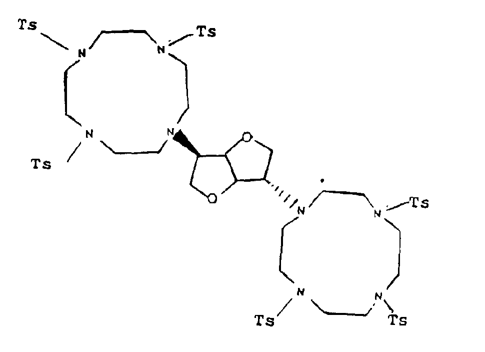

Dans un ballon tricol de 2 1 placé sous atmosphère d'argon sont introduits 15 g (16,1 mmoles) du composé obtenu à l'étape précédente, 750 cm³ de DMF sec et 32 g de Cs₂CO₃ anhydre. Une solution de 18,25 g (32,18 mmoles) de biséthanolamine tosylée dans 200 cm³ de DMF est ajoutée lentement. Le mélange est chauffé à 40° C pendant 3 jours. La solution est filtrée et le DMF est évaporé. Le composé est purifié sur colonne de silice.

8 g du produit du titre sont obtenus avec un rendement de 36 %.

CCM : Si0₂ CH₂CI₂/AcoEt: 90/10, Rf = 0,65.

RMN¹H : 7,4-7,8 ppm, multiplet : H aromatique, H des groupes tosyles, 2,4 ppm, singulet : méthyle, tosyle

### f) Préparation du composé de formule

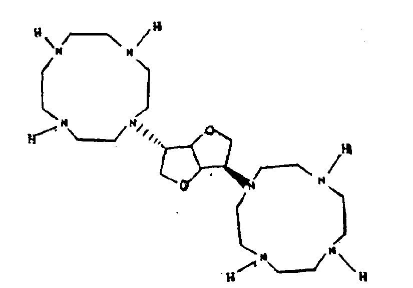

4 g (2,91 mmoles) de produit obtenu à l'étape précédente sont introduits dans 350 cm³ de n-butanol sec. le mélange est chauffé à 110° C sous agitation. Le réacteur est purgé à l'azote sec et 20 g de Na sont ajoutés par petites portions sur 8 heures.

La température est ensuite ramenée à 80° C et le mélange réactionnel est placé sous agitation pendant une nuit.

Après élimination complète du butanol, le résidu huileux est repris par HCI aqueux 1 M. La solution est filtrée et lavée au dichlorométhane.

Après évaporation, le chlorhydrate d'amine est récupéré sous forme d'un solide blanc.

2g de produit sont obtenus.

CCM : Si0₂; CH₂Cl₂/MeOH/NH₄ aqueux : 4/4/2, flamme ayant pour origine Rf = 0

RMN¹³C: 40,72 ppm - 48,42 ppm, H des macrocycles, 63,89 ppm - 66,03 ppm - 66,40 ppm - 70,16 ppm - 82,05 ppm - 83,25 ppm, motif bishétérocycle central.

### g) Préparation du composé de formule :

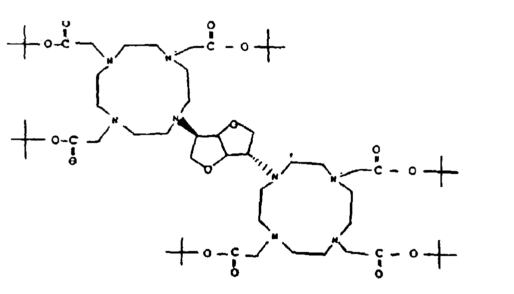

3,9 g de produit obtenu à l'étape précédente dans 45 cm³ d'acétonitrile sont introduits dans un ballon tricol de 500 cm³ muni d'un réfrigérant, d'un barreau magnétique et placé sous argon.

15 g de carbonate de sodium anhydre sont ajoutés avec 40 cm³ d'acétonitrile.

Le mélange est agité et porté à 40°C.

Une solution de 10,8 g de bromoacétate de terbutyle dans 30 cm³ d'acétonitrile est ensuite ajoutée goutte à goutte.

Le mélange est filtré après 24 heures et la solution réactionnelle est concentrée. L'huile obtenue est reprise à l'éther éthylique.

Le solide obtenu est lavé plusieurs fois à l'éther. Après purification sur colonne de silice, on obtient 5,6 g du composé du titre.
CCM:Si0₂; CH₂Cl₂/MeOH:90/10, Rf = 0,4
Spectre de masse FAB (glycérol) MH⁺ Na⁺ 1162

### h) Préparation du composé de formule :

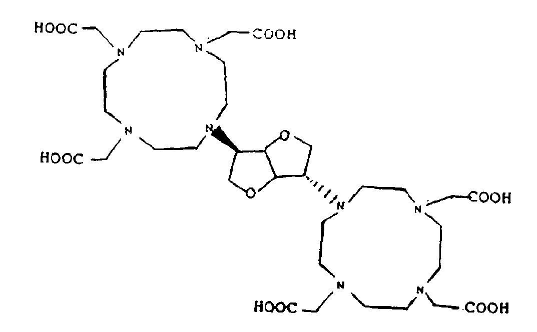

Le produit obtenu à l'étape précédente est dissous dans 30 cm³ d'acide trifluoroacétique.

Le milieu réactionnel est agité à température ambiante pendant 16 heures. Le solvant est évaporé et le résidu est repris à l'eau.

La solution obtenue est concentrée. Le produit est purifié sur résine IRA 458.

CCM:Si0₂; AcOEt/Isopropanol/ammoniaque aqueuse : 12/35/30 Rf = 0,25 + 0,42.

Spectre de masse FAB (glycérol), MH⁺ 803.
RMN¹³C : 171,9 ppm - 171,6 ppm - 167,4 ppm, carbonyles ;
57,55 ppm - 61,23 ppm - 62,57 ppm - 68,80 ppm - 79,18 ppm - 80,10 ppm : système hétérocyclique central, 53,9 ppm : méthylène des carboxyméthyles;
50,62 ppm - 41,64 ppm : macrocycles.

### EXEMPLE 4:

Préparation du composé de formule :

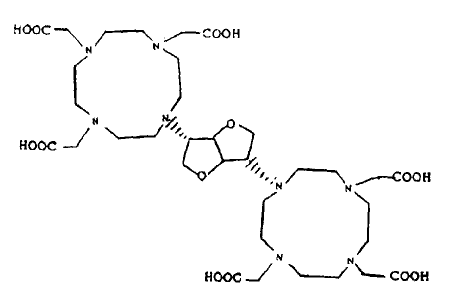

### a) Préparation du 2:5-ditosyl-1,4:3,6-dianhydromannitol de formule:

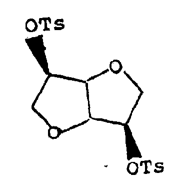

Le produit du titre est préparé à partir de 73 g (0,5 mole) de 1,4:3,6-dianhydro D-mannitol, disponible commercialement sous le nom Isomannide par la société Aldrich Chimie (Strasbourg) selon la méthode décrite dans Journal of Organometallic Chemistry 253 (1983) 249-252.

Masse obtenue = 196,2g ;

Rendement : 86,5%

### b) Préparation du 2:5-diazido-2,5-didéoxy--1,4:3,6-dianhydro-iditol.

Le composé du titre est préparé à partir de 98 g (0,2 mole) du composé obtenu à l'étape précédente selon la méthode décrite dans Carbohydrate Research 85 (1980) 259-269.

Masse obtenue : 36 g (rendement : 85%).

### c) Préparation du 2:5-diamino-2,5-didéoxy--1,4:3,6-dianhydro-iditol.

Le composé du titre est obtenu à partir de 12 g (0,06 mole) du composé de l'étape précédente selon les méthodes décrites dans JACS 78 (1956) 3180 et Synthetic Communications 19 (1989) 1493-1498.

Masse obtenue : 9g.

### d) Préparation du composé de formule :

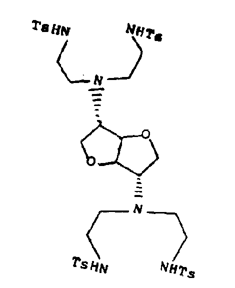

8,8 g (0,06 mole) du composé obtenu à l'étape précédente sont dissous dans 800 ml d'acétonitrile en présence de 48,15 g (0,244 mole) de N-tosylaziridine.

Le mélange réactionnel est chauffé au reflux pendant 48 h. Le milieu est évaporé à sec pour être ensuite chromatographié sur colonne. 26,1 g de produit sont ainsi obtenus soit un rendement de 51%.
CCM (CH₂Cl₂ 95/AcOEt 5/MeOH 5) : Rf = 0,5.
RMN¹H (200 MHz) (DMSO): 2,3 ppm, CH₃ tosyle 7,3 et 7,6 ppm, H aromatique.
RMN¹³C 128, 130, 138 et 143 ppm, C aromatique,
   85, 70, 68 ppm, C du système bishétérocyclique central,
   22 ppm, C du CH₃ du groupe tosyle.

### e) Préparation du composé de formule

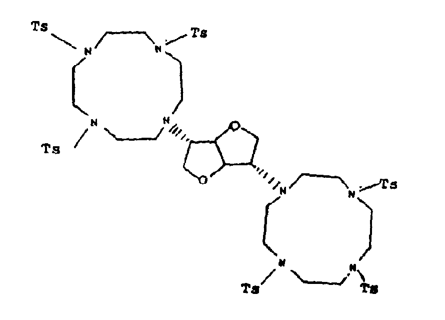

30 g du produit obtenu à l'étape précédente (0,0323 mole) sont dissous dans 1,8 1 de DMF anhydre en présence de 94 g de C_{S2}CO₃, sous argon, à40°C. Une heure plus tard, est ajouté lentement à cette suspension, une solution de 36,6 g (0,0645 mole) de diéthanolamine tritosylée dans 0,9 1 de DMF

Le milieu réaction est porté à 40°C pendant 48 h. Après filtration, évaporation du DMF, le résidu obtenu est chromatographié sur colonne.
16 g de produit sont ainsi obtenus, soit un rendement de 36%.
CCM:Si0₂; CH₂Cl₂ 95/AcoEt 4/MeOH 1 : Rf = 0,15.
I.R. (pastille de KBr) : disparition de la bande caractéristique de la liaison NH-Ts à 3300 cm⁻¹,
RMN¹³C (200 MHz) (DMSO) : 128, 130, 138 et 143 ppm, C aromatiques,
83,68 et 66 ppm, C du système bishétérocyclique central,
75 ppm et massif à 50 ppm, C des deux cycles formés,
22 ppm, CH₃ tosyl.

### f) Préparation du composé de formule :

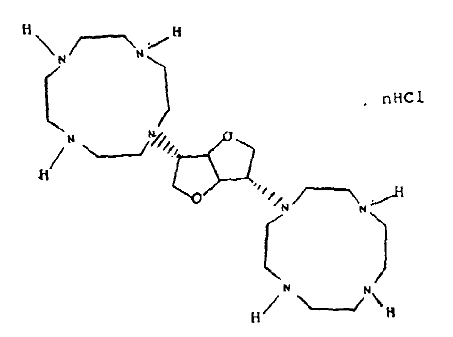

16 g (0,0116 mole) de produit obtenu à l'étape prcédente sont dissous dans 840 ml de n-butanol préalablement chauffé au reflux. A cette solution sont ajoutés lentement 73,6 g (3,2 moles) de sodium coupé en morceaux. Le milieu réactionnel est porté à 80°C pendant 12 h. Après élimination complète du solvant, le produit est repris dans 300 ml d'acide chlorhydrique. La solution obtenue est lavée 2 fois au CH₂CI₂. La phase aqueuse est évaporée à sec. On obtient 7,0 g de produit attendu soit un rendement de 90%.

CCM:Si0₂; CH₂Cl₂ 4/MeOH 4/NH₄0H 2 : Rf = 0,1 (révélation à l'I₂). RMN¹³C (DMSO) : 83,68 et 66 ppm, C du système hétérocyclique central, 44, 42, 38 et 37 ppm, C du bicycle.

### g) Préparation du composé de formule :

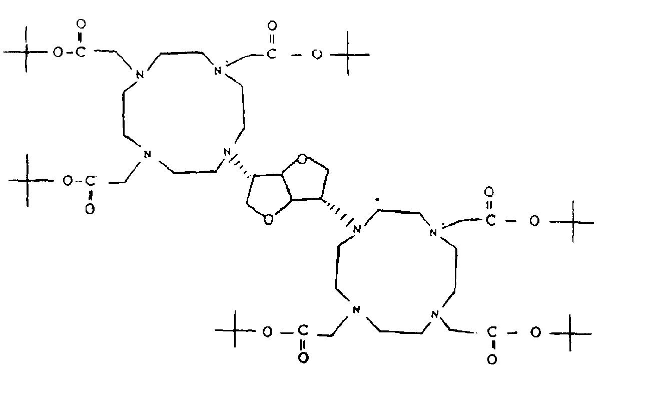

6 g (0,00886 mole) du produit obtenu à l'étape précédente sont mis en suspension dans 150 ml de CH₃CN à 40°C en présence de 18,8 g (0,177 mole) de Na₂CO₃. Au milieu est ajouté goutte à goutte une solution de 13,8 g (0,070 mole) de BrCH₂COOtBu dans 80 ml de CH₃CN. Le milieu réactionnel est chauffé à 40°C sous agitation pendant 72 heures. Après filtration, la solution est concentrée. Le produit attendu cristallise. Après filtration et lavage à l'éther, on obtient 8,3 g du produit attendu, soit un rendement de 77%.
CCM : Si0₂; CH₂Cl₂ 85/MeOH 15 : Rf = 0,6.
I.R. : 1710 cm⁻¹, bande carbonyle.
RMN¹H: 1,4 ppm singulet, groupement tertiobutyle.
RMN¹³C (DMSO) : 175 ppm, C du groupe carbonyle,
85 ppm, C quaternaire du groupe terbutyle,
27 ppm, CH₃,
63,68 et 80 ppm, système bishétérocyclique central.

### h) Préparation du composé de formule :

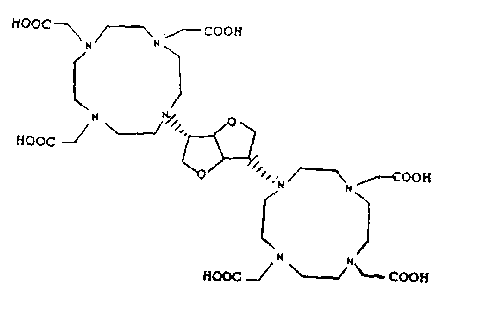

0,3 g (0,0073 môle) du produit obtenu à l'étape précédente sont dissous dans 130 ml de CF₃COOH, à température ambiante Après 12 h de réaction, le milieu réactionnel est concentré puis repris à l'eau et évaporé.

Le produit est purifié sur résine IRA 458.
CCM : SiO₂; AcOEt 12/isopropanol 35/NH₄0H 30 : Rf = 0,2 + 0,35.
RMN¹³C:167 et 172 ppm, C acide,
63, 68 et 80 ppm, système hétérocyclique central,
54 ppm, CH₂ en a de COOH,
Multiplet de 40 < 8 <50 ppm, C des deux cycles.
Spectre de masse FAB (glycérol), MH⁺ 803.

### EXEMPLE 5

Préparation du composé de formule :

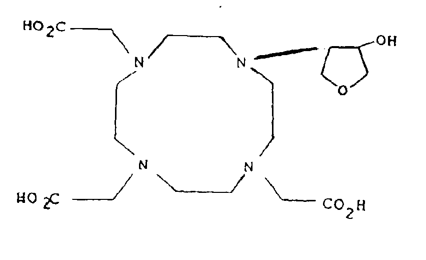

### a) Préparation du 3,4-époxy tétrahydrofuranne

Le produit du titre est obtenu à partir de 58 g (0,83 mole) de 2,5-dihydrofuranne disponible commercialement auprès de la société Aldrich Chimie (Strasbourg) selon la méthode décrite dans Journal of Pharmaceutical Sciences 59 (1970) 1676-1679.

Masse obtenue : 54 g Rendement : 71 %.

### b) Préparation du 3-hydroxy 4-azido tétrahydrofuranne.

Le produit du titre est obtenu à partir de 54 g (0,63 mole) du produit obtenu à l'étape précédente selon la méthode décrite dans Tetrahedron Letters 3 (1990) 5641-5644.

Masse obtenue : 73 g; rendement 90%.

### c) Préparation du 3-hydroxy 4-amino tétrahydrofuranne.

Le composé du titre est obtenu à partir de 24 g (0,19 mole) du produit de l'étape précédente selon la méthode décrite dans Synthetis 4 (1990) 366-368.

Masse obtenue : 20 g

### d) Préparation du composé de formule :

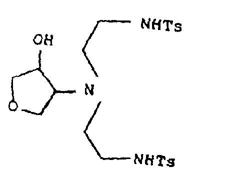

10 g (0,097 mole) du produit de l'étape précédente sont mélangés à 38,2 g (0,19 mole) de N-tosylaziridine dans 600 ml d'acétonitrile à 50°C pendant 72 heures. La solution est concentrée puis le
résidu obtenu est cristallisé dans un mélange acétate d'éthyle/toluène.

Après filtration, lavage et séchage, 31 g de produit sont obtenus, soit un rendement de 64%.
CCM : Si0₂; CH₂Cl₂ 90/MeOH 10 : Rf - 0,5.
RMN¹H (200 MHz) (DMSO) : 2 doublets à 7,4 et 7,7 ppm, protons aromatiques,
5 ppm, OH,
protons de l'hétérocycle : singulet à 4 ppm, multiplet à 3,7 ppm, multiplet à 3,4 ppm,
2< δ <3 ppm, CH₂-CH₂ et CH₃ (Tₛ).

### e) Préparation du composé de formule :

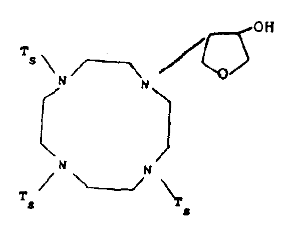

18 g (0,0362 mole) du produit de l'étape précédente sont mis en solution dans 1200 ml de DMF en présence de 54 g (0,165 mole) de carbonate de césium, à une température de 40°C, sous argon.

Une solution de 20,6 g (0,0363 mole) de diéthanolamine tritosylée est ajoutée dans 600 ml de DMF

Après la fin de la réaction, le milieu réactionnel est filtré puis concentré.

Le résidu est chromatographié sur colonne.
15 g de produit sont obtenus, rendement : 58%.
CCM : SiO₂; CH₂Cl₂ 95/MeOH 5 : Rf = 0,2.
I.R. (KBr) 3300 cm⁻¹, NHTs.
RMN¹H (DMSO) Protons aromatiques :
   7,6 ppm triplet,
   7,4 ppm doublet,
   5,1 ppm, doublet, OH;
   Protons du tétrahydrofuranne : 4,1 ppm, singulet,
   2 < 8 < 3,9 ppm massif.

### f) Préparation du composé de formule :

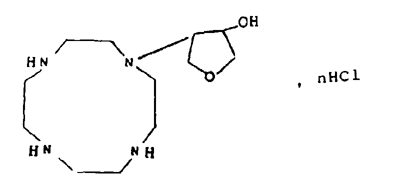

20,5 g (0,028 mole) du produit de l'étape précédente sont dissous dans 440 ml de n-butanol au reflux.

32,0 g (1,4 mole) de sodium en copeaux sont ajoutés lentement. Le milieu réactionnel est ensuite porté à une température de 80°C, sous agitation.

Après élimination du butanol à l'eau, le résidu est repris dans 300 ml d'HCl 1 N. La solution obtenue est lavée au CH₂Cl₂ puis évaporé à sec.
9 g de produit sont obtenus;
CCM : SiO₂; CH₂Cl₂ 4/MeOH 4/NH₄0H 2 : Rf = 0,1 (1₂)
RMN¹³C : 84-72-69 et 67 ppm, C du tétrahydrofuranne;
45 et 42 ppm, C cycle.

### g) Préparation du composé de formule :

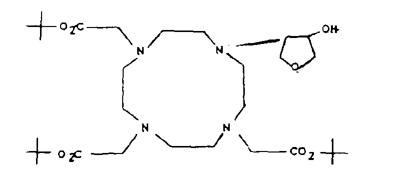

54 g (0,147 mole) du produit de l'étape précédente sont mis en suspension dans 1,5 1 d'acétonitrile, en présence de 155,5 g (1,46 mole) de Na₂CO₃, à une température de 40°C.

Une solution de 114,5 g (0,6 mole) de bromoacétate de terbutyle dans 0,7 1 d'acétonitrile est ajoutée lentement. Le milieu réactionnel est ensuite filtré et évaporé à sec. Le résidu obtenu est cristallisé dans l'éther éthylique puis chromatographié sur colonne. 69 g du produit sont obtenus. Rendement : 72%.
CMM : Si0₂; CH₂Cl₂ 80/MeOH 20 : Rf = 0,6.
I.R. (KBr) 1710 cm⁻¹, C = O.
RMN¹³C : 170 et 173 ppm, C carbonyle,
81 ppm, C quaternaire,
84, 72, 69 et 67 ppm, C tétrahydrofuranne,
55 ppm, C du CH₂ en a de l'ester,
52, 49 et 46 ppm, C du cycle,
28 ppm, CH₃ du terbutyle.

### h) Préparation du composé de formule:

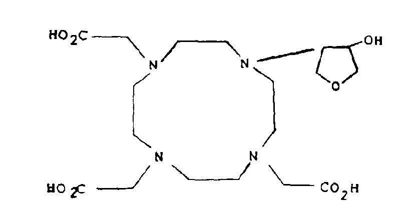

38 g (0,063 mole) du produit de l'étape précédente sont mis en solution dans 600 ml d'acide trifluoroacétique à température ambiante.

Après 18 heures, le milieu réactionnel est concentré à sec puis repris à l'eau. Après purification sur résine IRA 458, 20 g de produit sont obtenus. Rendement : 73%.

CCM:Si0₂; AcOEt 12/Isopropanol 35/NH₄OH 30 : Rf = 0,45 et 0,5.
RMN¹³C : 170 ppm, 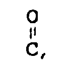
73, 72, 69 et 67 ppm, C du tétrahydrofuranne,
55 ppm, C de CH₃ en a du COOH,
55 ppm < δ <45 ppm, C du cycle.
Spectre de masse : FAB (glycérol) MH⁺ 433.

### EXEMPLE 6:

Préparation du composé de formule

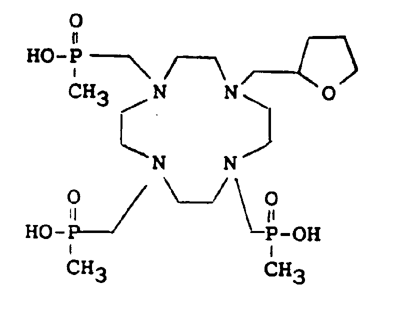

### a) Préparation du composé de formule :

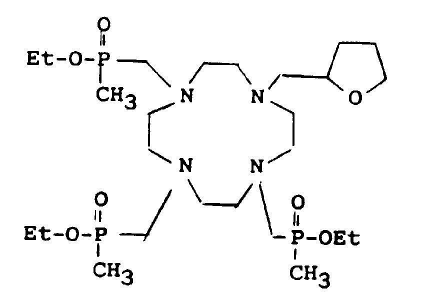

0,7 g (2,7 mmoles) du composé obtenu à l'étape c) de l'exemple 1 ci-dessus, 0,7 g de paraformaldéhyde et 2,5 g (16,2 mmoles) de diéthylméthyl-phosphite obtenu selon la méthode décrite dans Organic Préparations and Procedures Int. 11 (1) 11-16 (1979) dans 30 ml de THF sont chauffés au reflux pendant 12 heures.

Après évaporation, la solution est chromatographiée sur alumine en éluant un dichlorométhane.

0,5 g d'une huile jaune pâle sont obtenus, soit un rendement de 30%.
CCM:Si0₂; CH₂Cl₂/MeOH : 90/10;
Rf = 0,2.

### b) Préparation du composé de formule :

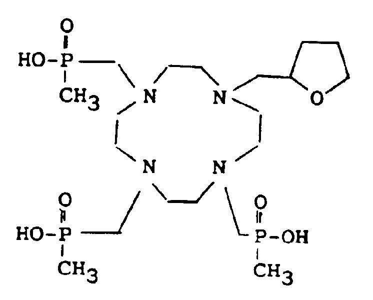

0,5 g (0,8 mmoles) du composé obtenu à l'étape précédente dans 25 ml d'HCI 6N sont chauffés au reflux pendant 12 heures.

Après évaporation, le produit est purifié sur silice silanisée.

0,25 g du composé du titre sont obtenus, soit un rendement de 42%.
CCM:SiO₂; dioxanne/eau/NH3 : 8/3/2;
Rf : 0,1.

## Revendications (Revendications pour l'(les) Etat(s) contractant(s) suivant(s) : AT, BE, CH, DE, DK, FR, GB, GR, IT, LI, LU, MC, NL, PT, SE)

1. Ligands de formule II: 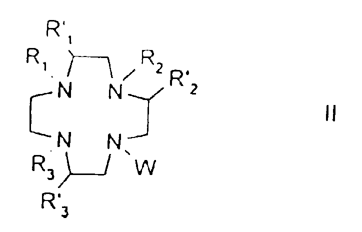 dans laquelle
R₁, R₂, R₃ identiques ou différents sont choisis parmi un atome d'hydrogène, et les groupes 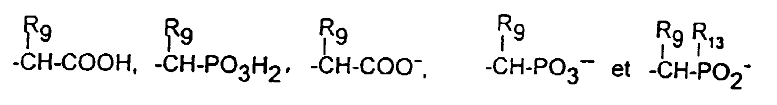
R₉ représentant un atome d'hydrogène, un groupe alkyle à chaîne linéaire ou ramifiée en C₁-C₆, hydroxyalkyle à chaîne linéaire ou ramifiée en C₁-C₆, polyhydroxyalkyle à chaîne linéaire ou ramifiée en C₁-C₆, alkoxy (en C₁-C₆) alkyle en C₁-C₆ à chaîne linéaire ou ramifiée, R₁₃ représentant un groupe alkyle à chaîne linéaire ou ramifiée en C₁-C₆, un groupe hydroxy- ou polyhydroxyalkyle à chaîne linéaire ou ramifiée en C₁-C₆, un groupe alkoxy (en C₁-C₆) alkyle à chaîne linéaire ou ramifiée en C₁-C₆, un groupe hydroxy- ou polyhydroxyalkoxy (en C₁-C₆) alkyle en chaîne linéaire ou ramifiée en C₁-C₆, sous réserve qu'au moins deux groupes parmi R₁, R₂ ou R₃ représentent 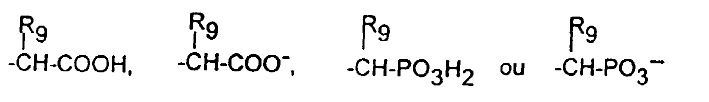
R'₁, R'₂ ou R'₃, identiques ou différents représentant un atome d'hydrogène, un groupe alkyle à chaîne linéaire ou ramifiée en C₁-C₆, un groupe hydroxyalkyle ou polyhydroxyalkyle à chaîne linéaire ou ramifiée en C₁-C₆, W est choisi parmi les groupes suivants : 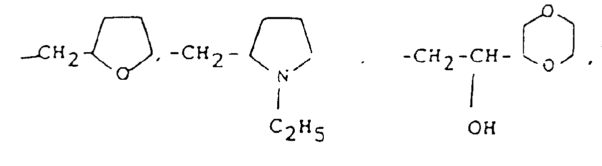 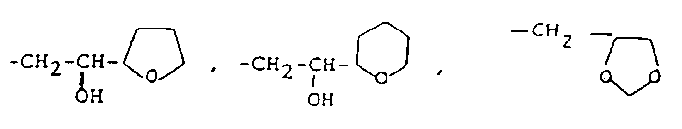 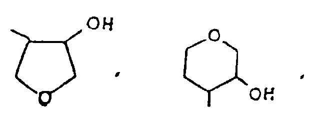 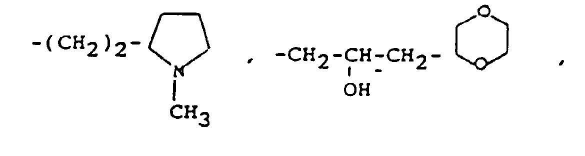 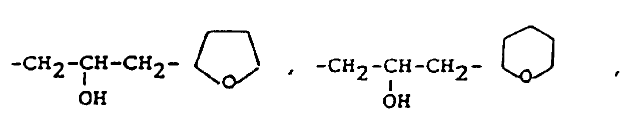 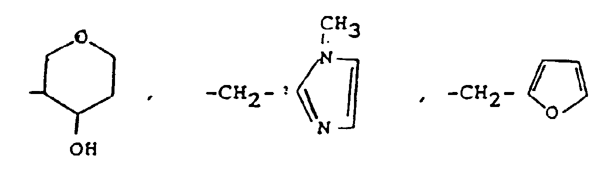 et 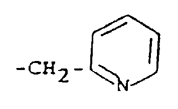 ou W représente un groupe 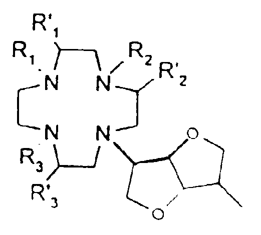 dans lequel R₁, R₂, R₃, R'₁, R'₂ et R'₃ sont tels que définis précédemment, ainsi que les sels de ces composés formés avec des bases minérales en organiques, ou des aminoacides basiques.

2. Ligands selon la revendication 1 de formule III 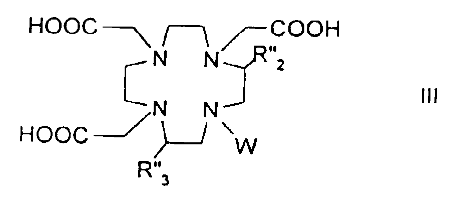 dans laquelle R"₂ et R"₃ sont choisis parmi un atome d'hydrogène, le groupe méthyle et le groupe éthyle et W représente le groupe

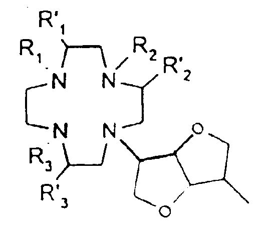

3. Ligands selon la revendication 1 de formule III 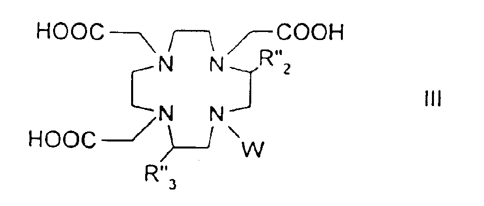 dans laquelle R"₂ et R"₃ représentent un atome d'hydrogène et W représente les groupes:

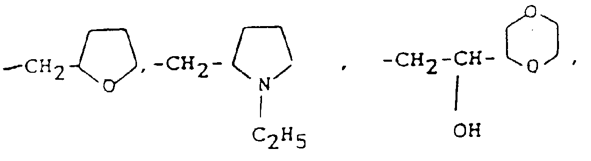

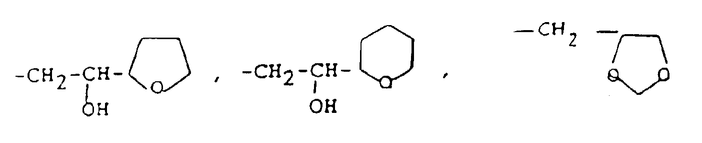

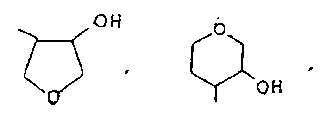

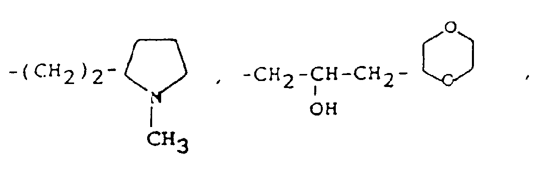

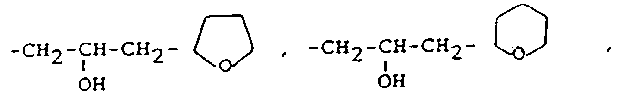

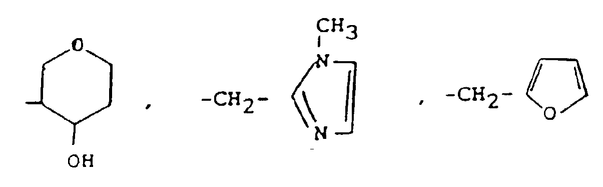

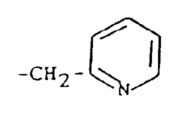

4. Ligand de formule

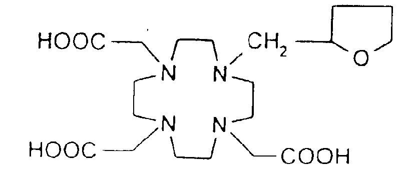

5. Ligand selon la revendication 1 de formule III 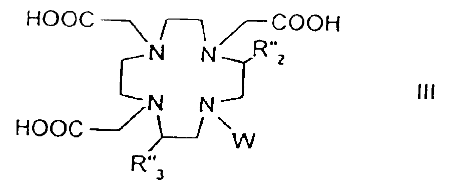 dans laquelle R"₂ et R"₃ représentent le groupe méthyle et W représente

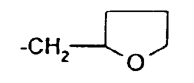

6. Ligand de formule

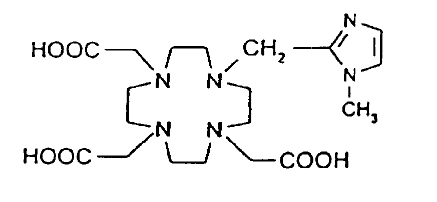

7. Ligand de formule

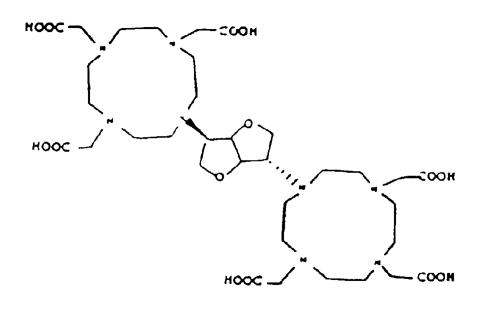

8. Ligand de formule

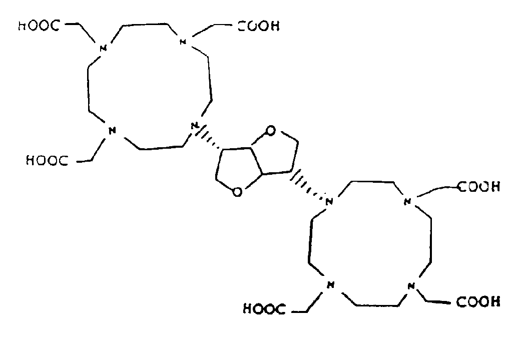

9. Ligand de formule

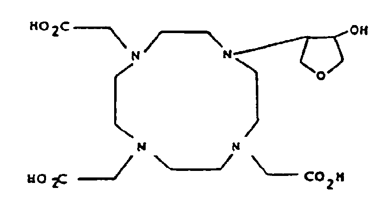

10. Procédé de préparation des composés de formule générale II selon la revendication 1, caractérisé par les étapes suivantes:
a) réaction d'un composé de formule 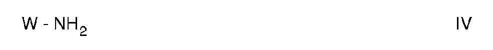 dans laquelle W est tel que défini à la revendication 1 avec un composé de formule 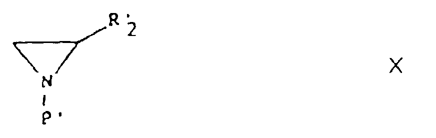 dans lequel R'₂ est tel que défini à la revendication 1 et P' représente un groupe tosyle, mésyle, phénylsulfonyle, ou 4-méthoxyphénylsulfonyle, pour obtenir un composé de formule XI 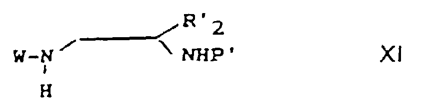
b) réaction du composé de formule XI avec un composé de formule XII 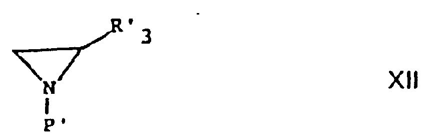 R'₃ étant tel que défini dans la revendication 1 et P' étant tel que défini précédemment pour obtenir le composé de formule XIII 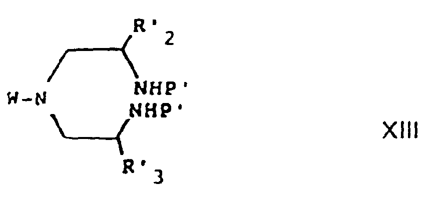
c) réaction du composé de formule XIII avec un composé de formule 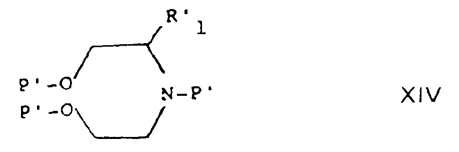 P' étant tel que défini précédemment et R'₁ étant tel que défini dans la revendication 1 pour obtenir un composé de formule XV 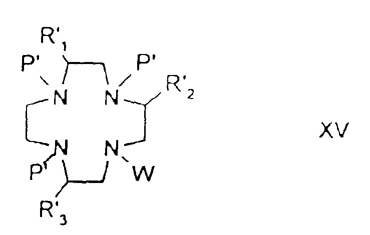
d) élimination du groupe P', et
e) alkylation du composé obtenu pour obtenir un composé de formule II.

11. Complexes mono- ou polymétalliques neutres ou ioniques formés par au moins un ligand de formule II selon la revendication 1, avec au moins un ion métallique choisi parmi les ions des lanthanides, les ions des métaux de transition, du baryum, du bismuth, du plomb et des radioisotopes ^{99m}Tc, ¹¹¹In, ⁹⁰Y, ⁶⁴Cu et ¹⁶⁹Yb, ainsi que les sels de ces complexes avec des bases minérales ou organiques pharmaceutiquement acceptables ou des aminoacides basiques.

12. Complexes mono- ou bimétalliques selon la revendication 11, dans lesquels l'ion métallique est choisi parmi le gadolinium, l'europium, le dysprosium, le fer (Fe³⁺), le manganèse (Mn²⁺) et le baryum.

13. Composition de diagnostic pour l'imagerie par résonance magnétique caractérisée en ce qu'elle comprend au moins un complexe selon l'une quelconque des revendications 11 à 12.

## Revendications (Revendications pour l'(les) Etat(s) contractant(s) suivant(s) : ES)

1. Procédé de préparation de composés de formule générale II: 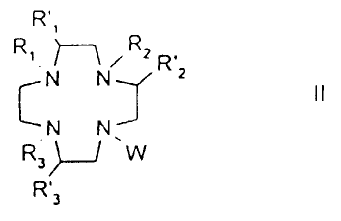 dans laquelle
R₁, R₂, R₃ identiques ou différents sont choisis parmi un atome d'hydrogène, et les groupes 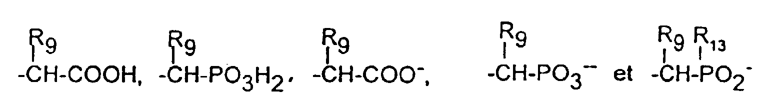
R₉ représentant un atome d'hydrogène, un groupe alkyle à chaîne linéaire ou ramifiée en C₁-C₆, hydroxyalkyle à chaîne linéaire ou ramifiée en C₁-C₆, polyhydroxyalkyle à chaîne linéaire ou ramifiée en C₁-C₆, alkoxy (en C₁-C₆) alkyle en C₁-C₆ à chaîne linéaire ou ramifiée, R₁₃ représentant un groupe alkyle à chaîne linéaire ou ramifiée en C₁-C₆, un groupe hydroxy- ou polyhydroxyalkyle à chaîne linéaire ou ramifiée en C₁-C₅, un groupe alkoxy (en C₁-C₆) alkyle à chaîne linéaire ou ramifiée en C₁-C₆, un groupe hydroxy- ou polyhydroxyalkoxy (en C₁-C₆) alkyle en chaîne linéaire ou ramifiée en C₁-C₆, sous réserve qu'au moins deux groupes parmi R₁, R₂ ou R₃ représentent 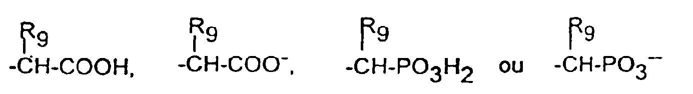
R'₁, R'₂ ou R'₃, identiques ou différents représentant un atome d'hydrogène, un groupe alkyle à chaîne linéaire ou ramifiée en C₁-C₆, un groupe hydroxyalkyle ou polvhvdroxvalkvle à chaîne linéaire ou ramifiée en C₁-C₆, W est choisi parmi les groupes suivants : 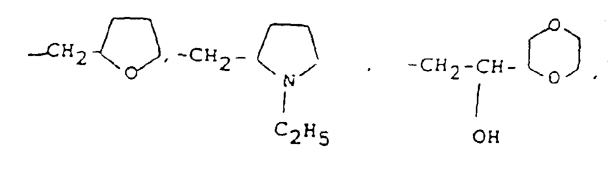 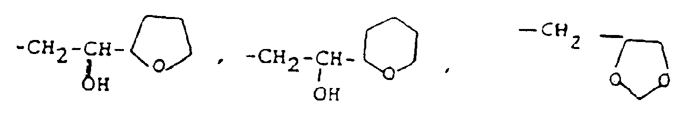 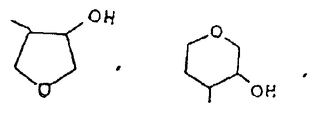 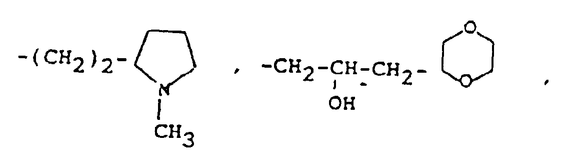 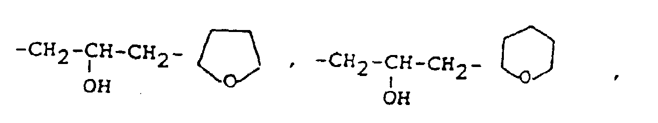 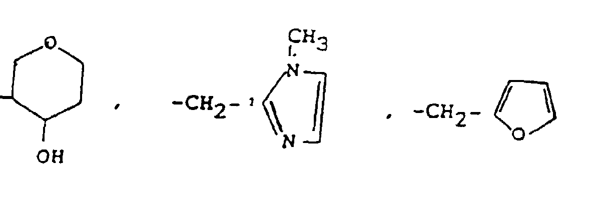 et 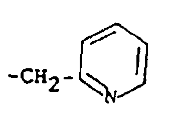 ou W représente un groupe : 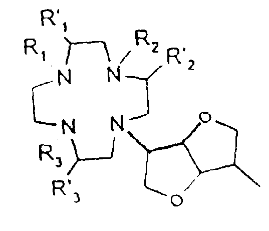 dans lequel R₁, R₂, R₃, R'₁, R'₂ et R'₃ sont tels que définis précédemment caractérisé par les étapes suivantes :
a) réaction d'un composé de formule 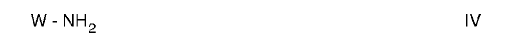 dans laquelle W est tel que défini à la revendication 1 avec un composé de formule 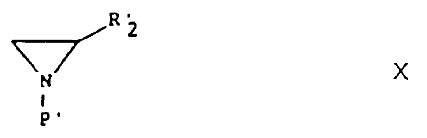 dans lequel R'₂ est tel que défini à la revendication 1 et P' représente un groupe tosyle, mésyle, phénylsulfonyle, ou 4-méthoxyphénylsulfonyle, pour obtenir un composé de formule XI 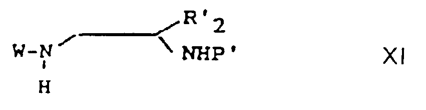
b) réaction du composé de formule XI avec un composé de formule XII 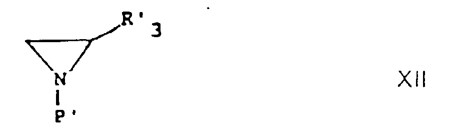 R'₃ étant tel que défini dans la revendication 1 et P' étant tel que défini précédemment pour obtenir le composé de formule XIII 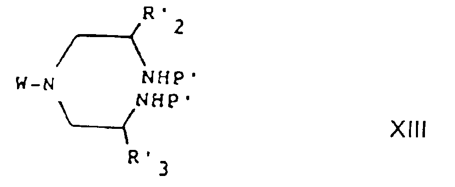
c) réaction du composé de formule XIII avec un composé de formule 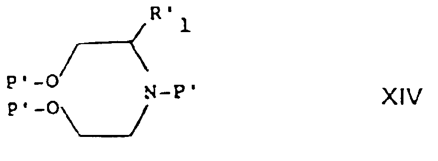 P' étant tel que défini précédemment et R'₁ étant tel que défini dans la revendication 1 pour obtenir un composé de formule XV 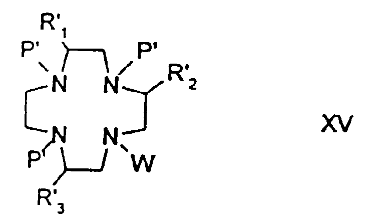
d) élimination du groupe P', et
e) alkylation du composé obtenu pour obtenir un composé de formule II.

2. Procédé selon la revendication 1 pour la préparation d'un ligand de formule III : 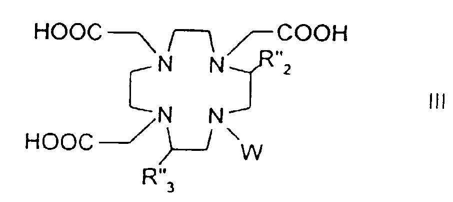 dans laquelle R"₂ et R"₃ sont choisis parmi un atome d'hydrogène, le groupe méthyle et le groupe éthyle et W représente le groupe:

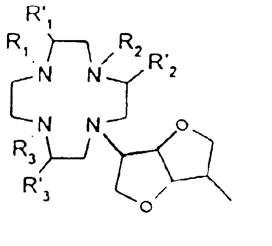

3. Procédé selon la revendication 1 pour la préparation d'un ligand de formule III : 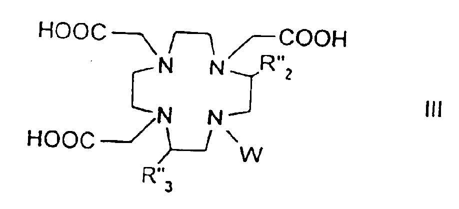 dans laquelle R"₂ et R"₃ représentent un atome d'hydrogène et W représente les groupes : 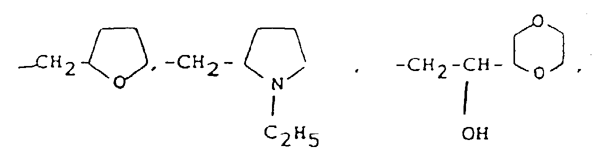 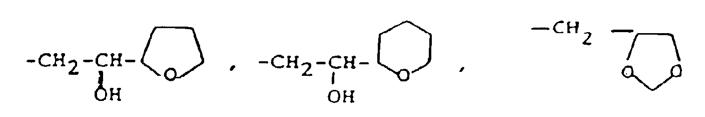 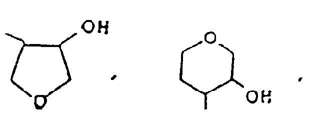 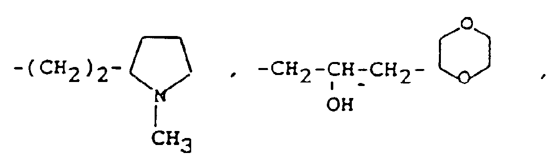 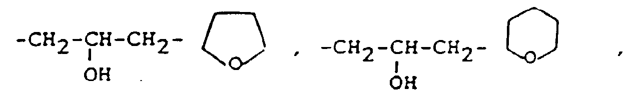 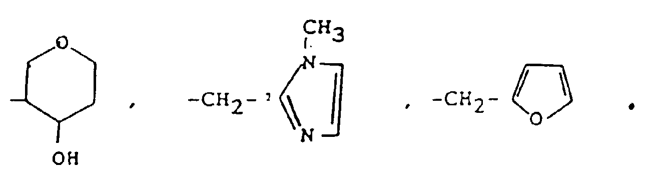 ou

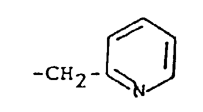

4. Procédé selon la revendication 1 pour la préparation de composés de formule :

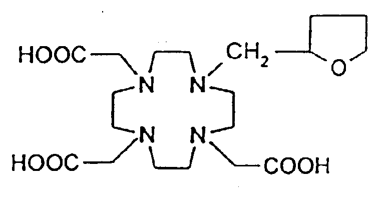

5. Procédé selon la revendication 1 pour la préparation de composés de formule III : 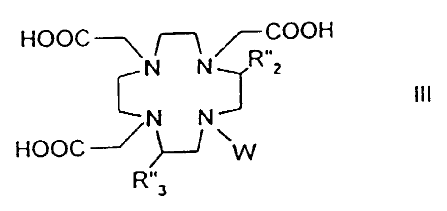 dans laquelle R"₂ et R"₃ représentent le groupe méthyle et W représente :

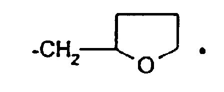

6. Procédé selon la revendication 1 pour la préparation de composés de formule III :

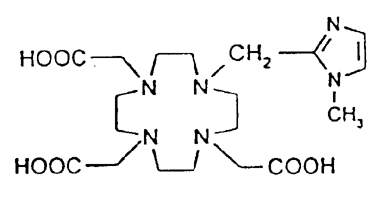

7. Procédé selon la revendication 1 pour la préparation de composés de formule

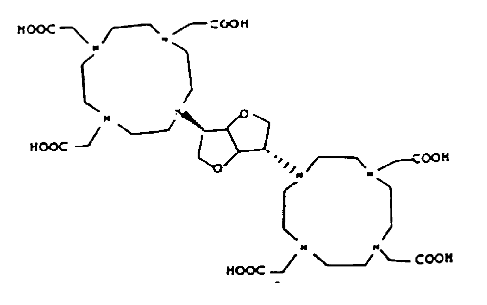

8. Procédé selon la revendication 1 pour la préparation de composés de formule

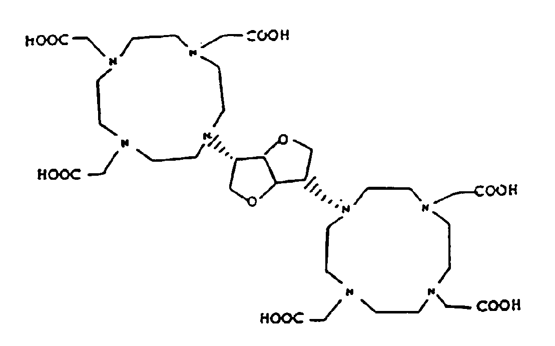

9. Procédé selon la revendication 1 pour la préparation de composés de formule

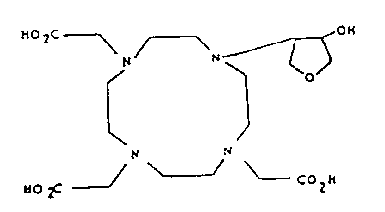

10. Procédé pour la préparation de complexes mono- ou polymétalliques neutres ou ioniques formés par au moins un ligand de formule Il selon la revendication 1, avec au moins un ion métallique choisi parmi les ions des lanthanides, les ions des métaux de transition, du baryum, du bismuth, du plomb et des radioisotopes ^{99m}Tc, ¹¹¹In, ⁹⁰Y, ⁶⁴Cu et ¹⁶⁹Yb, ainsi que de leurs sels avec des bases minérales ou organiques pharmaceutiquement acceptables ou des aminoacides basiques, caractérisé en ce que l'on fait réagir un ligand obtenu par le procédé d'une des revendications 1 à 9 avec un sel ou un oxyde du métal correspondant et, le cas échéant on neutralise le mélange résultant pour l'obtention d'un sel.

11. Procédé selon la revendication 10 pour la préparation de complexes mono- ou bimétalliques, dans lesquels l'ion métallique est choisi parmi le gadolinium, l'europium, le dysprosium, le fer (Fe³⁺), le manganèse (Mn²⁺) et le baryum.

12. Ligands de formule II. 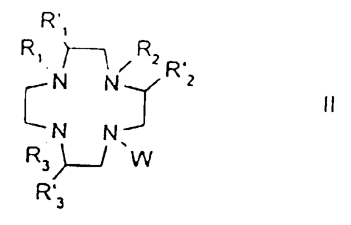 dans laquelle
R₁, R₂, R₃ identiques ou différents sont choisis parmi un atome d'hydrogène, et les groupes 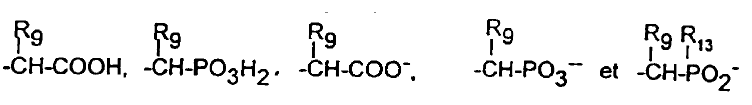
R₉ représentant un atome d'hydrogène, un groupe alkyle à chaîne linéaire ou ramifiée en C₁-C₆, hydroxyalkyle à chaîne linéaire ou ramifiée en C₁-C₆, polyhydroxyalkyle à chaîne linéaire ou ramifiée en C₁-C_{6;} alkoxy (en C₁-C₆) alkyle en C₁-C₆ à chaîne linéaire ou ramifiée, R₁₃ représentant un groupe alkyle à chaîne linéaire ou ramifiée en C₁-C₆, un groupe hydroxy- ou polyhydroxyalkyle à chaîne linéaire où ramifiée en C₁-C₆, un groupe alkoxy (en C₁-C₆) alkyle à chaîne linéaire ou ramifiée en C₁-C₆, un groupe hydroxy- ou polyhydroxyalkoxy (en C₁-C₆) alkyle en chaîne linéaire ou ramifiée en C₁-C_{6'} sous réserve qu'au moins deux groupes parmi R_{1'} R₂ ou R₃ représentent 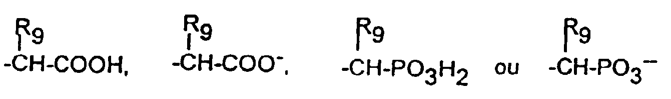
R'_{1'} R'₂ ou R'₃, identiques ou différents représentant un atome d'hydrogène, un groupe alkyle à chaîne linéaire ou ramifiée en C₁-C₆, un groupe hydroxyalkyle ou polyhydroxyalkyle à chaîne linéaire ou ramifiée en C₁-C₆, W est choisi parmi les groupes suivants : 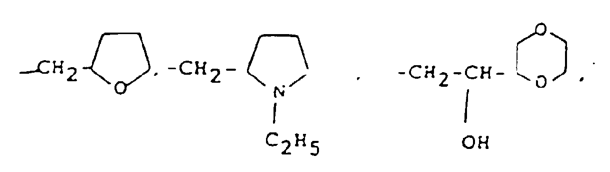 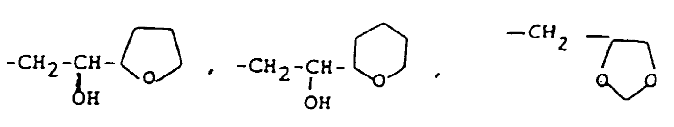 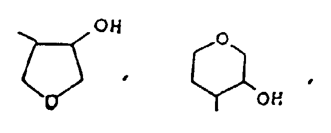 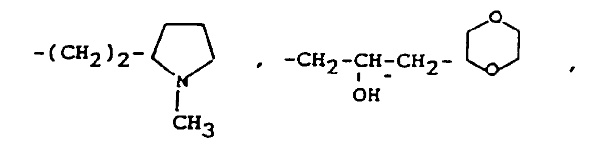 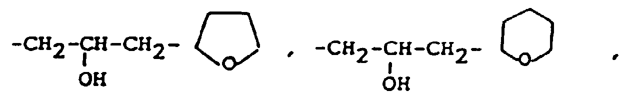 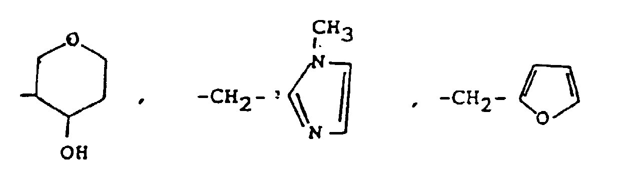 et 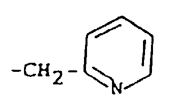 ou W représente un groupe 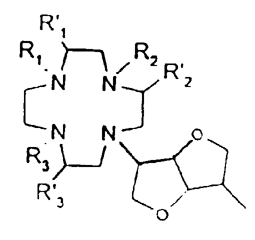 dans lequel R_{1'} R₂, R₃, R'₁, R'₂ et R'₃ sont tels que définis précédemment, ainsi que les sels de ces composés formés avec des bases minérales en organiques, ou des aminoacides basiques.

## Patentansprüche (Patentansprüche für folgende(n) Vertragsstaat(en) : AT, BE, CH, DE, DK, FR, GB, GR, IT, LI, LU, MC, NL, PT, SE)

1. Liganden der Formel II: 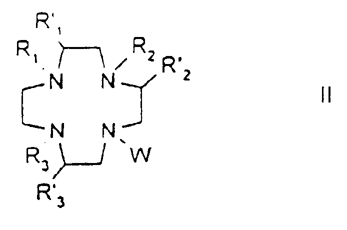 in der
R_{1'} R₂ und R₃, die gleichartig oder verschieden sind, ausgewählt sind aus einem Wasserstoffatom und Gruppen der Formeln 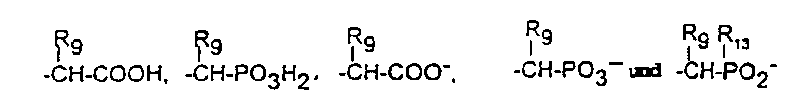 in denen R₉ ein Wasserstoffatom, eine geradkettige oder verzweigte C₁-C₆-Alkylgruppe, eine geradkettige oder verzweigte C₁-C₆-Hydroxylalkylgruppe, eine geradkettige oder verzweigte C₁-C₆-Polyhydroxyalkylgruppe, eine geradkettige oder verzweigte C₁-C₆-Alkoxy-(C₁-C₆)-alkylgruppe; R₁₃ eine geradkettige oder verzweigte C₁-C₆-Alkylgruppe, eine geradkettige oder verzweigte C₁-C₆-Hydroxy- oder -Polyhydroxyalkylgruppe, eine geradkettige oder verzweigte C₁-C₆-Alkoxy-(C₁-C₆)-alkylgruppe, eine geradkettige oder verzweigte (C₁-C₆)-Hydroxy-oder-Polyhydroxyalkoxy-(C₁-C₆)-alkylgruppe mit der Maßgabe darstellen, daß mindestens zwei der Gruppen R₁, R₂ oder R₃ Gruppen der Formel 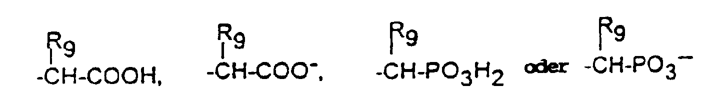 darstellen,
R'₁, R'₂ oder R'₃, die gleichartig oder verschieden sind, ein Wasserstoffatom, eine geradkettige oder verzweigte C₁-C₆-Alkylgruppe oder eine geradkettige oder verzweigte C₁-C₆-Hydroxyalkyl- oder-Polyhydroxyalkylgruppe darstellen, W aus den folgenden Gruppen ausgewählt ist: 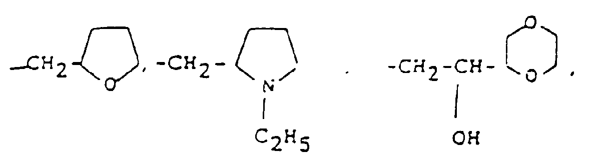 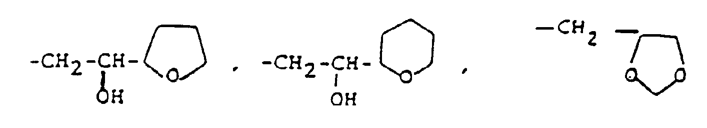 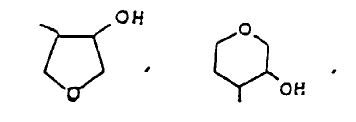 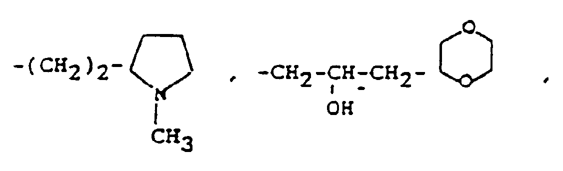 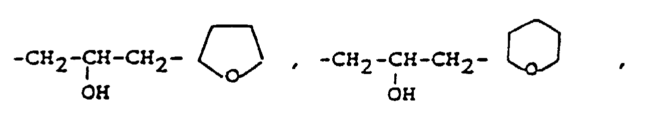 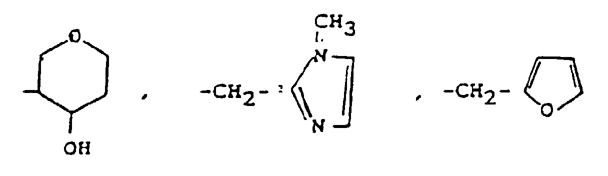 und 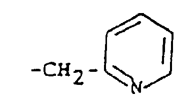 oder W eine Gruppe der Formel 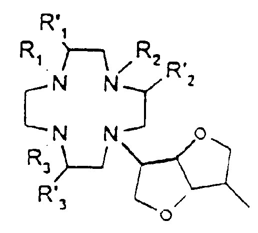 darstellt;
in der R₁, R₂, R₃, R'₁, R'₂ und R'₃ die oben angegebenen Bedeutungen besitzen, sowie die Salze dieser Verbindungen mit anorganischen oder organischen Basen oder basischen Aminosäuren.

2. Liganden nach Anspruch 1 der Formel III 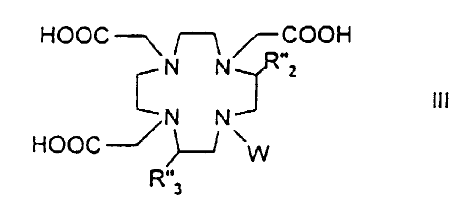 in der R"₂ und R"₃ aus Wasserstoffatomen, Methylgruppen und Ethylgruppen ausgewählt sind und W die Gruppe der Formel 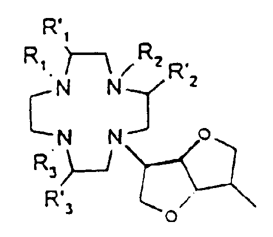 darstellt.

3. Liganden nach Anspruch 1 der Formel III 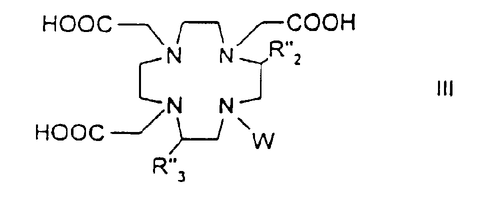 in der R"₂ und R"₃ ein Wasserstoffatom und W eine der Gruppen bedeuten:

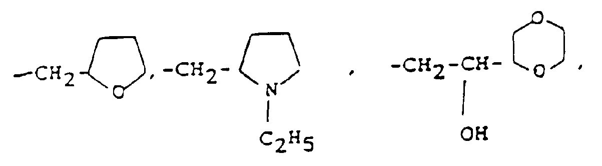

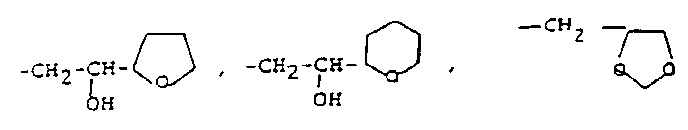

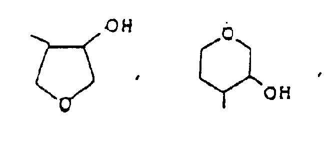

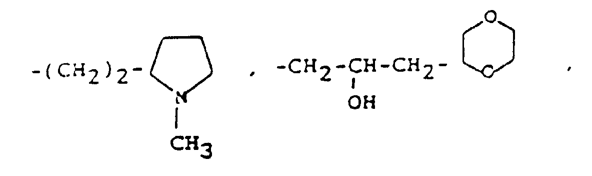

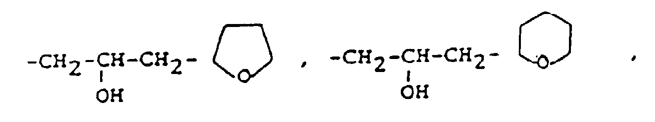

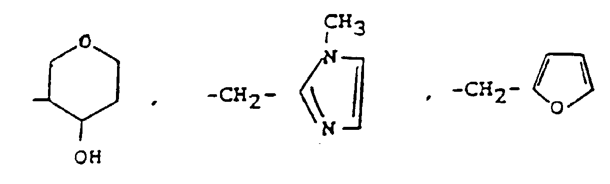

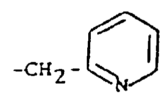

4. Ligand der Formel

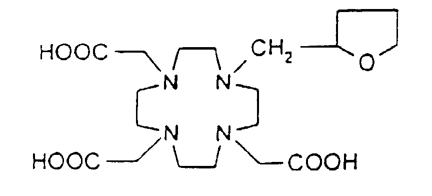

5. Ligand nach Anspruch 1 der Formel III 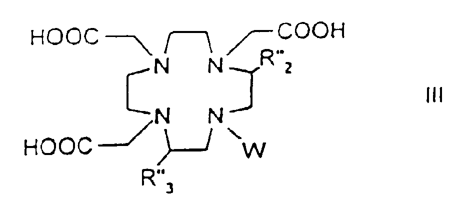 in der R"₂ und R"₃ eine Methylgruppe und W eine Gruppe der Formel 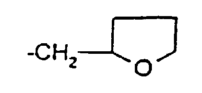 bedeuten.

6. Ligand der Formel

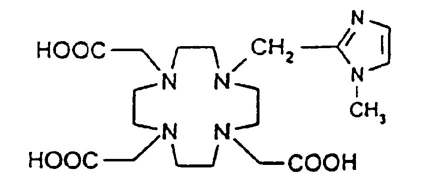

7. Ligand der Formel

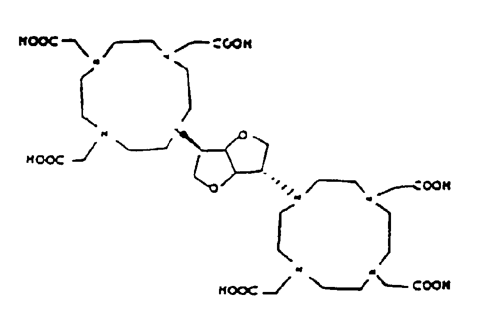

8. Ligand der Formel

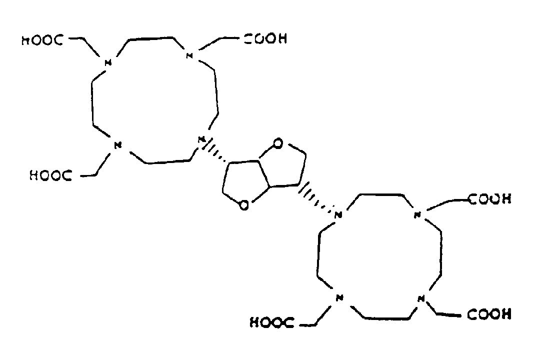

9. Ligand der Formel

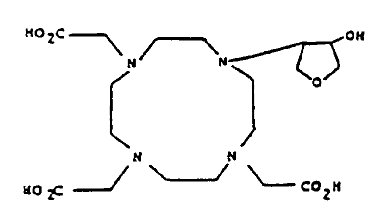

10. Verfahren zur Herstellung der Verbindungen der allgemeinen Formel II nach Anspruch 1, gekennzeichnet durch die folgenden Stufen:
a) Umsetzung einer Verbindung der Formel 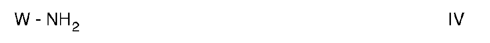 in der W die in Anspruch 1 angegebenen Bedeutungen besitzt, mit einer Verbindung der Formel 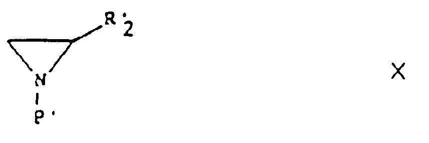 in der R'₂ die in Anspruch 1 angegebenen Bedeutungen besitzt und P' für eine Tosyl-, Mesyl-, Phenylsulfonyl-oder 4-Methoxyphenylsulfonylgruppe steht zur Bildung einer Verbindung der Formel XI 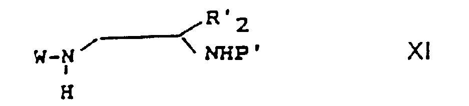
b) Umsetzung der Verbindung der Formel XI mit einer Verbindung der Formel XII 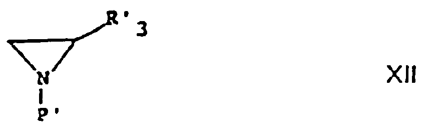 in der R'₃ die in Anspruch 1 angegebenen Bedeutungen und P' die oben angegebenen Bedeutungen besitzen zur Bildung der Verbindung der Formel XIII 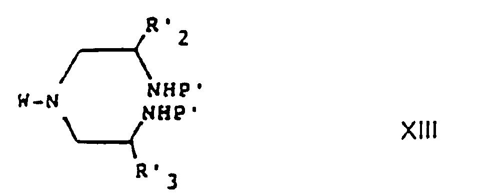
c) Umsetzung der Verbindung der Formel XIII mit einer Verbindung der Formel 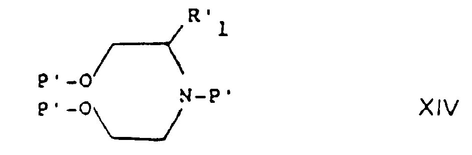 in der P' die oben angegebenen Bedeutungen und R'₁ die in Anspruch 1 angegebenen Bedeutungen besitzen zur Bildung einer Verbindung der Formel XV 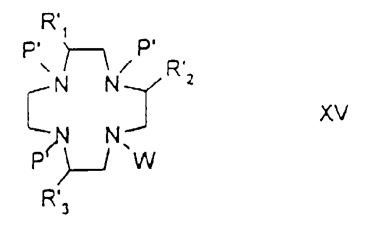
d) Eliminierung der Gruppe P' und
e) Alkylierung der erhaltenen Verbindung zur Bildung einer Verbindung der Formel II.

11. Mono-oder polymetallische neutrale oder ionische Komplexe gebildet aus mindestens einem Liganden der Formel II nach Anspruch 1 mit mindestens einem Metallion ausgewählt aus Ionen der Lanthanide. Ionen der Übergangsmetalle, von Barium. Bismut, Blei und den Radioisotopen ^{99m}Tc, ¹¹¹In, ⁹⁰Y; ⁶⁴C_{U} und ¹⁶⁹Yb, sowie die Salze dieser Verbindungen mit pharmazeutisch annehmbaren anorganischen oder organischen Basen oder basischen Aminosäuren.

12. Mono- oder Bimetall-Komplexe nach Anspruch 11, worin das Metallion aus Gadolinium, Europium, Dysprosium, Eisen (Fe³⁺); Mangan (Mn²⁺) und Barium ausgewählt ist.

13. Zubereitung für die Bilddiagnose durch magnetische Resonanz, dadurch gekennzeichnet, daß sie mindestens einen Komplex nach einem der Ansprüche 11 bis 12 umfaßt.

## Patentansprüche (Patentansprüche für folgende(n) Vertragsstaat(en) : ES)

1. Verfahren zur Herstellung der Verbindungen der allgemeinen Formel 11: 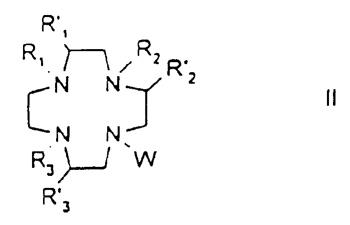 in der
R₁, R₂ und R₃, die gleichartig oder verschieden sind. ausgewählt sind aus einem Wasserstoffatom und Gruppen der Formeln 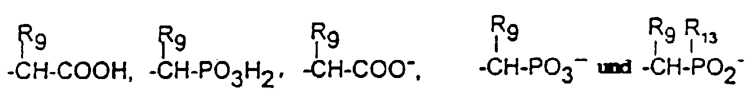 in denen R₉ ein Wasserstoffatom, eine geradkettige oder verzweigte C₁-C₆-Alkylgruppe, eine geradkettige oder verzweigte C₁-C₆-Hydroxylalkylgruppe, eine geradkettige oder verzweigte C₁-C₆-Polyhydroxyalkylgruppe, eine geradkettige oder verzweigte C₁-C₆-Alkoxy-(C₁-C₆)-alkylgruppe, R₁₃ eine geradkettige oder verzweigte C₁-C₆-Alkylgruppe, eine geradkettige oder verzweigte C₁-C₆-Hydroxy- oder -Polyhydroxyalkylgruppe, eine geradkettige oder verzweigte C₁-C₆-Alkoxy-(C₁-C₆)-alkylgruppe, eine geradkettige oder verzweigte (C₁-C₆)-Hydroxy-oder-Polyhydroxyalkoxy-(C₁-C₆)-alkylgruppe mit der Maßgabe darstellen, daß mindestens zwei der Gruppen R₁, R₂ oder R₃ Gruppen der Formel 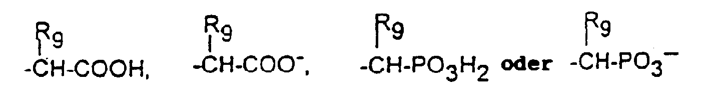 darstellen,
R'₁, R'₂ oder R'₃, die gleichartig oder verschieden sind, ein Wasserstoffatom, eine geradkettige oder verzweigte C₁-C₆-Alkylgruppe oder eine geradkettige oder verzweigte C₁-C₆-Hydroxyalkyl- oder-Polyhydroxyalkylgruppe darstellen. W aus den folgenden Gruppen ausgewählt ist: 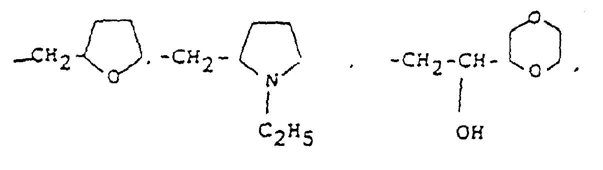 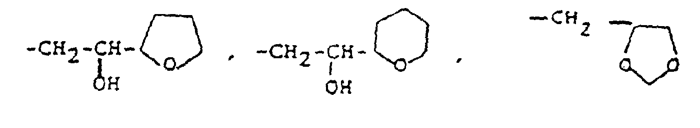 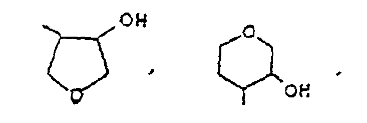 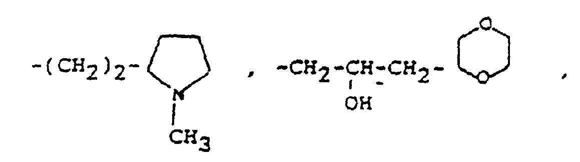 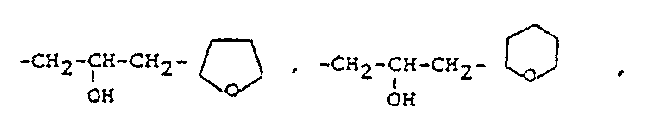 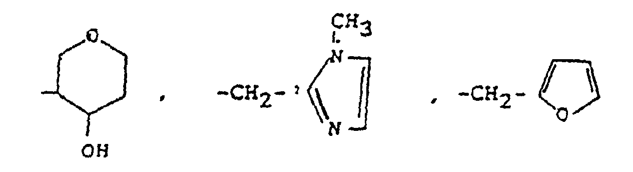 und 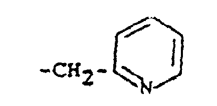 oder W eine Gruppe der Formel 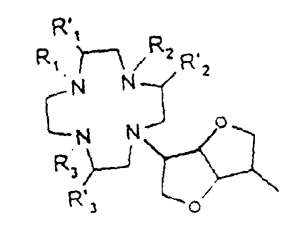 darstellt.
in der R₁, R₂, R₃, R'₁, R'₂ und R'₃ die oben angegebenen Bedeutungen besitzen, gekennzeichnet durch die folgenden Stufen:
a) Umsetzung einer Verbindung der Formel 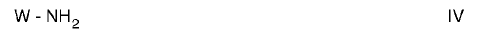 in der W die in Anspruch 1 angegebenen Bedeutungen besitzt, mit einer Verbindung der Formel 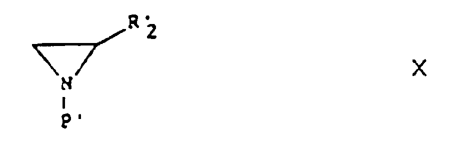 in der R'₂ die in Anspruch 1 angegebenen Bedeutungen besitzt und P' für eine Tosyl-, Mesyl-, Phenylsulfonyl-oder 4-Methoxyphenylsulfonylgruppe steht zur Bildung einer Verbindung der Formel XI 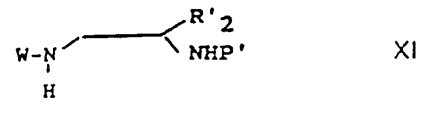
b) Umsetzung der Verbindung der Formel XI mit einer Verbindung der Formel XII 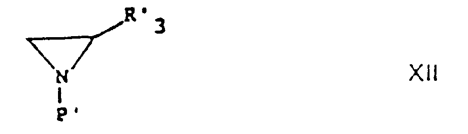 in der R'₃ die in Anspruch 1 angegebenen Bedeutungen und P' die oben angegebenen Bedeutungen besitzen zur Bildung der Verbindung der Formel XIII 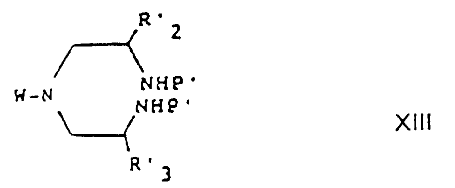
c) Umsetzung der Verbindung der Formel XIII mit einer Verbindung der Formel 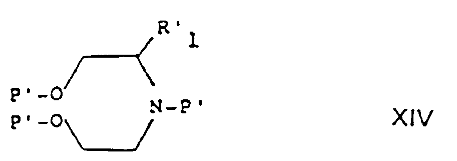 in der P' die oben angegebenen Bedeutungen und R'₁ die in Anspruch 1 angegebenen Bedeutungen besitzen zur Bildung einer Verbindung der Formel XV 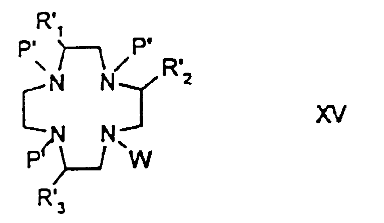
d) Eliminierung der Gruppe P' und
e) Alkylierung der erhaltenen Verbindung zur Bildung einer Verbindung der Formel II.

2. Verfahren nach Anspruch 1 für die Herstellung eines Liganden der Formel III: 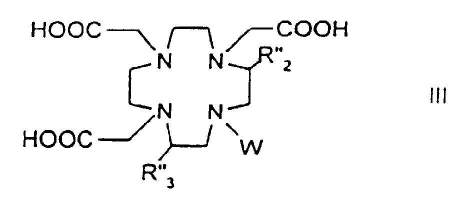 in der R"₂ und R"₃ aus Wasserstoffatomen, Methylgruppen und Ethylgruppen ausgewählt sind und W die Gruppe der Formel 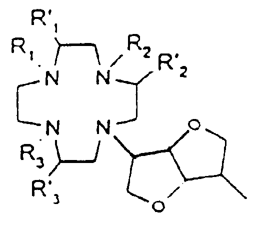 darstellt.

3. Verfahren nach Anspruch 1 zur Herstellung eines Liganden der Formel III: 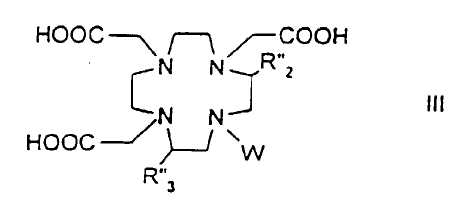 in der R"₂ und R"₃ ein Wasserstoffatom und W eine der Gruppen bedeuten: 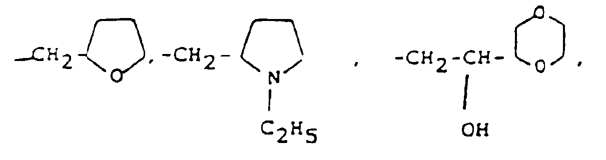 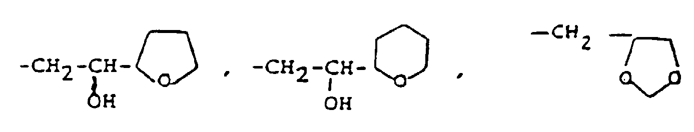 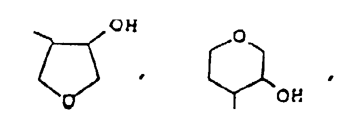 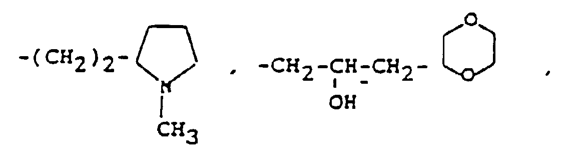 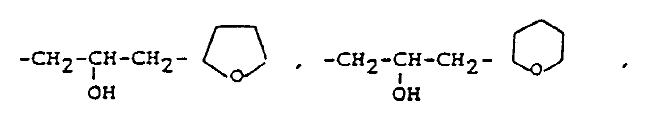 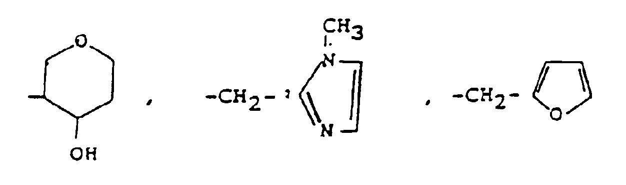 oder

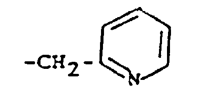

4. Verfahren nach Anspruch 1 zur Herstellung der Verbindungen der Formel:

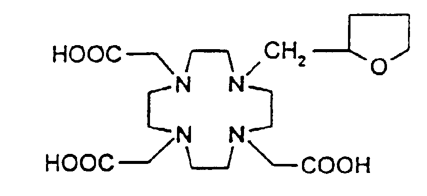

5. Verfahren nach Anspruch 1 zur Herstellung der Verbindung der Formel III: 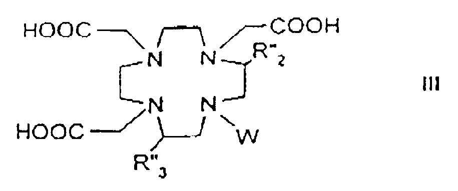 in der R"₂ und R"₃ eine Methylgruppe und W eine Gruppe der Formel 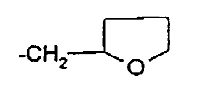 bedeuten.

6. Verfahren nach Anspruch 1 zur Herstellung der Verbindungen der Formel

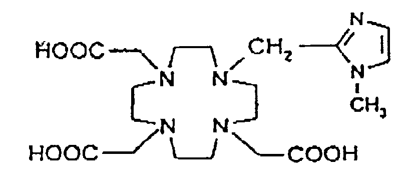

7. Verfahren nach Anspruch 1 zur Herstellung der Verbindungen der Formel

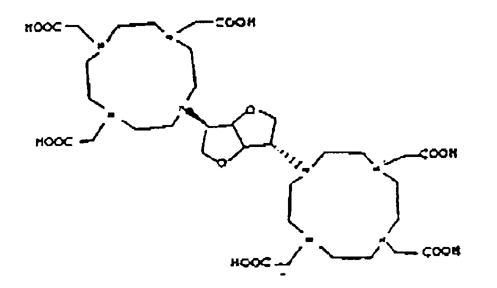

8. Verfahren nach Anspruch 1 zur Herstellung der Verbindungen der Formel

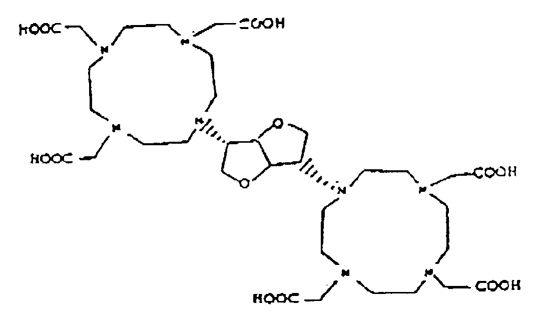

9. Verfahren nach Anspruch 1 zur Herstellung der Verbindungen der Formel

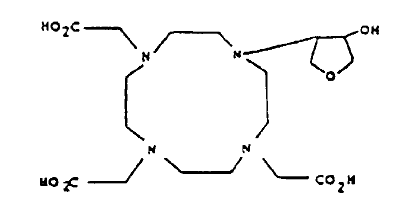

10. Verfahren zur Herstellung von mono- oder polymetallischen neutralen oder ionischen Komplexen, die aus mindestens einem Liganden der Formel II nach Anspruch 1 mit mindestens einem Metallion ausgewählt aus Ionen der Lanthanide, Ionen der Übergangsmetalle. von Barium, Bismut, Blei und den Radioisotopen ^{99m}Tc, ¹¹¹In, ^{90y}, ⁶⁴C_{U} und ¹⁶⁹Yb, sowie von deren Salzen mit anorganischen oder organischen, pharmazeutisch annehmbaren Basen oder basischen Aminosäuren, dadurch gekennzeichnet. daß man einen nach einem Verfahren der Ansprüche 1 bis 9 erhaltenen Liganden mit einem Salz oder einem Oxid des entsprechenden Metalls umsetzt und gegebenenfalls die erhaltene Mischung zur Bildung eines Salzes neutralisiert.

11. Verfahren nach Anspruch 10 zur Herstellung von mono- oder bimetallischen Komplexen, in denen das Metallion aus Gadolinium, Europium, Dysprosium, Eisen (Fe³⁺); Mangan (Mn²⁺) und Barium ausgewählt ist.

12. Liganden der Formel II: 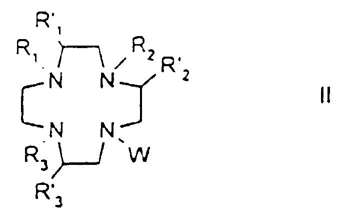 in der
R₁, R₂ und R₃, die gleichartig oder verschieden sind. ausgewählt sind aus einem Wasserstoffatom und Gruppen der Formeln 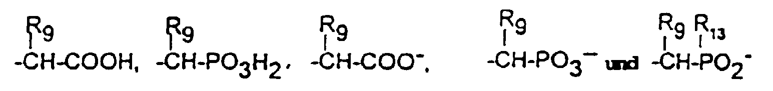 in denen R₉ ein Wasserstoffatom, eine geradkettige oder verzweigte C₁-C₆-Alkylgruppe, eine geradkettige oder verzweigte C₁-C₆-Hydroxylalkylgruppe, eine geradkettige oder verzweigte C₁-C₆-Polyhydroxyalkylgruppe, eine geradkettige oder verzweigte C₁-C₆-Alkoxy-(C₁-C₆)-alkylgruppe, R₁₃ eine geradkettige oder verzweigte C₁-C₆-Alkylgruppe, eine geradkettige oder verzweigte C₁-C₆-Hydroxy- oder -Polyhydroxyalkylgruppe, eine geradkettige oder verzweigte C₁-C₆-Alkoxy-(C₁-C₆)-alkylgruppe, eine geradkettige oder verzweigte (C₁-C₆)-Hydroxy-oder-Polyhydroxyalkoxy-(C₁-C₆)-alkylgruppe mit der Maßgabe darstellen, daß mindestens zwei der Gruppen R₁, R₂ oder R₃ Gruppen der Formel 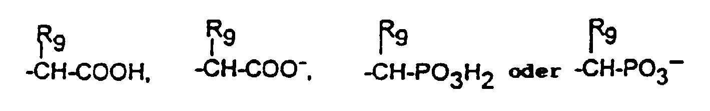 darstellen,
R'₁, R'₂ oder R'₃, die gleichartig oder verschieden sind, ein Wasserstoffatom, eine geradkettige oder verzweigte C₁-C₆-Alkylgruppe oder eine geradkettige oder verzweigte C₁-C₆-Hydroxyalkyl- oder-Polyhydroxyalkylgruppe darstellen, W aus den folgenden Gruppen ausgewählt ist: 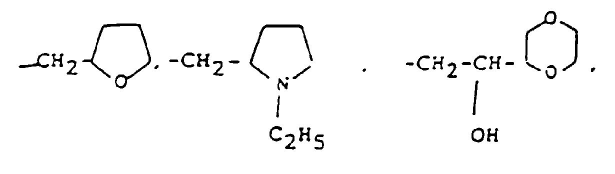 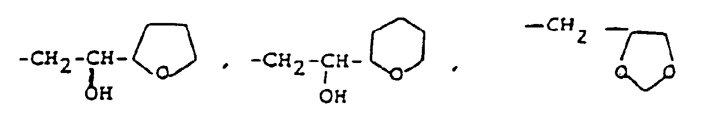 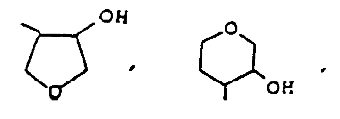 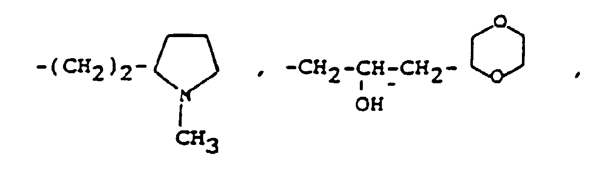 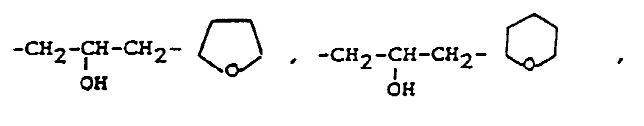 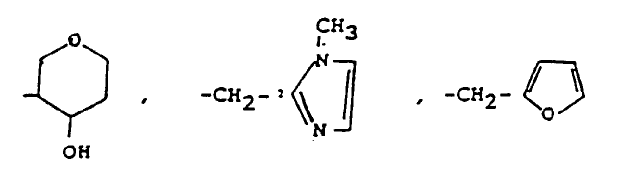 und 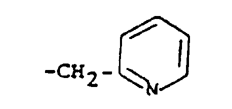 oder W eine Gruppe der Formel 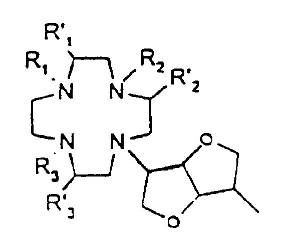 darstellt.
in der R₁, R₂, R₃, R'₁, R'₂ und R'₃ die oben angegebenen Bedeutungen besitzen, sowie die Salze dieser Verbindungen mit anorganischen oder organischen Basen oder basischen Aminosäuren.

## Claims (Claims for the following Contracting State(s) : AT, BE, CH, DE, DK, FR, GB, GR, IT, LI, LU, MC, NL, PT, SE)

1. Ligands of formula II: 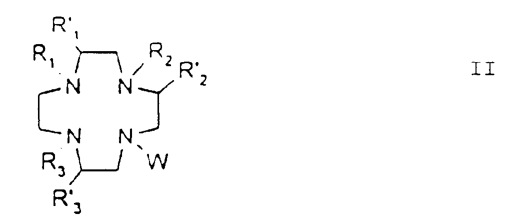 wherein R₁, R₂, R₃, which are the same or different, are selected from a hydrogen atom and the groups 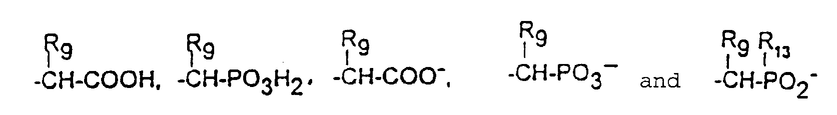 R₉ representing a hydrogen atom, a C₁-C₆ linear or branched chain alkyl group, a C₁-C₆ linear or branched chain hydroxyalkyl group, a C₁-C₆ linear or branched chain polyhydroxyalkyl group, or a C₁-C₆ linear or branched chain (C₁-C₆ alkoxy)alkyl group, and R₁₃ representing a C₁-C₆ linear or branched chain alkyl group, a C₁-C₆ linear or branched chain hydroxy- or polyhydroxyalkyl group, a C₁-C₆ linear or branched chain (C₁-C₆ alkoxy)alkyl group, or a C₁-C₆ linear or branched chain (C₁-C₆ hydroxy- or polyhydroxyalkoxy)alkyl group, with the proviso that at least two groups from among R₁, R₂ and R₃ represent 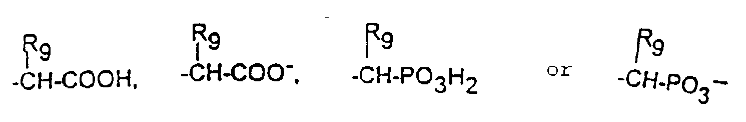 R'₁, R'₂ or R'₃, which are the same or different, representing a hydrogen atom, a C₁-C₆ linear or branched chain alkyl group, or a C₁-C₆ linear or branched chain hydroxyalkyl or polyhydroxyalkyl group, W is selected from the following groups: 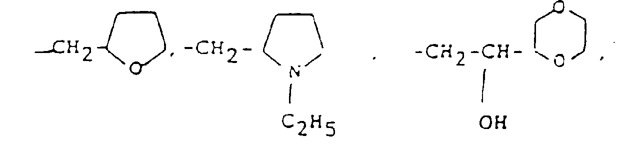 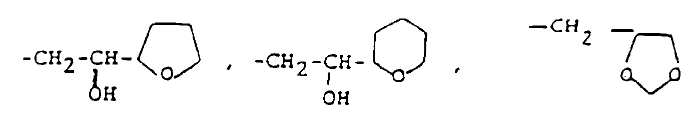 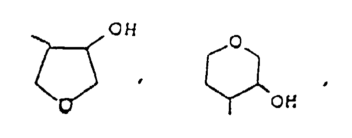 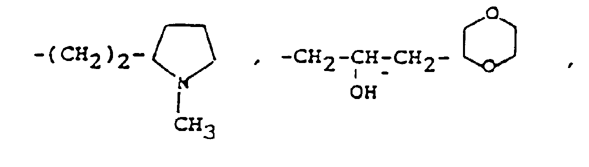 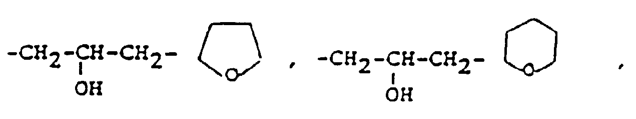 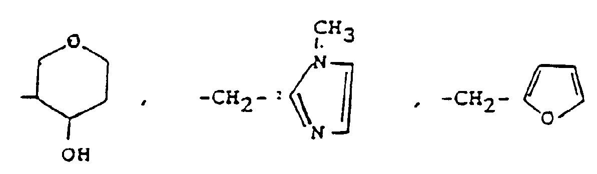 and 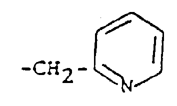 or W represents a group 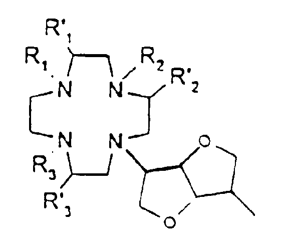 wherein R₁, R₂, R₃, R'₁, R'₂ and R'₃ are as defined above,
and the salts of those compounds formed with mineral or organic bases or with basic amino acids.

2. Ligands according to claim 1 of formula III 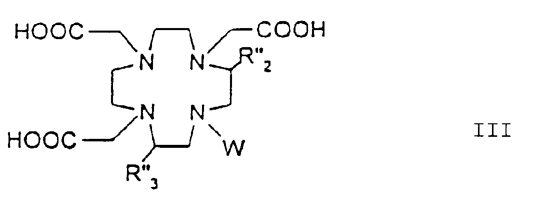 wherein R"₂ and R"₃ are selected from a hydrogen atom, the methyl group and the ethyl group and W represents the group

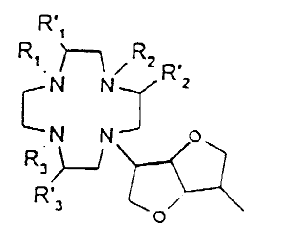

3. Ligands according to claim 1 of formula III 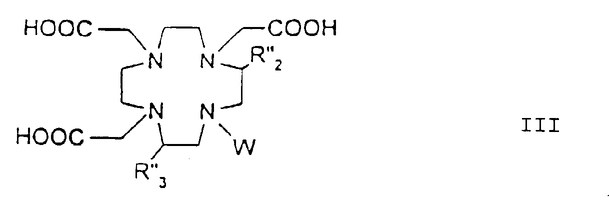 wherein R"₂ and R"₃ represent a hydrogen atom and W represents the groups:

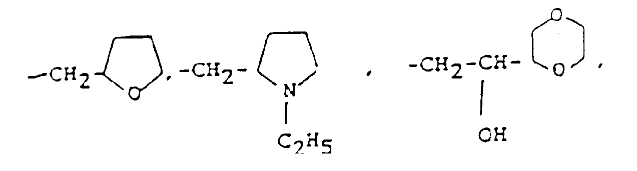

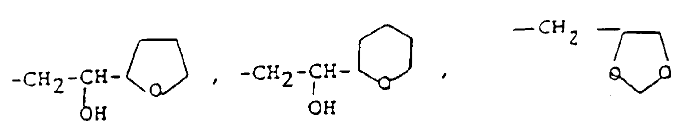

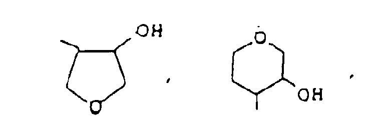

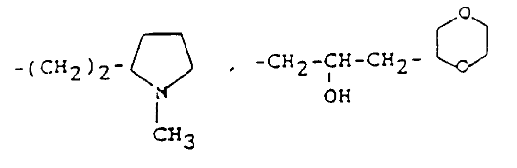

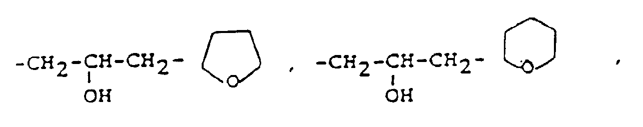

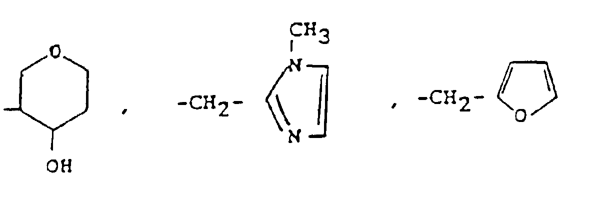

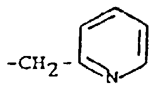

4. Ligand of formula

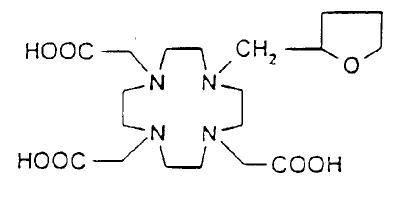

5. Ligand according to claim 1 of formula II 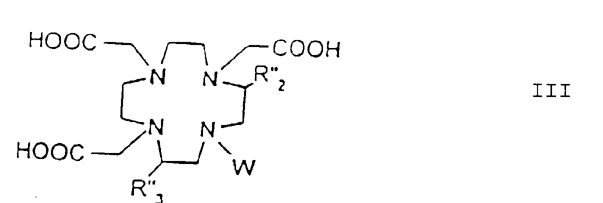 wherein R"₂ and R"₃ represent the methyl group and W represents

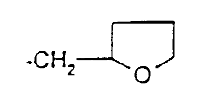

6. Ligand of formula

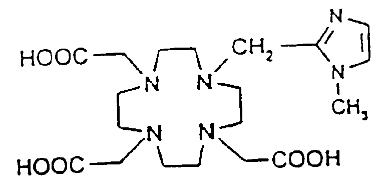

7. Ligand of formula

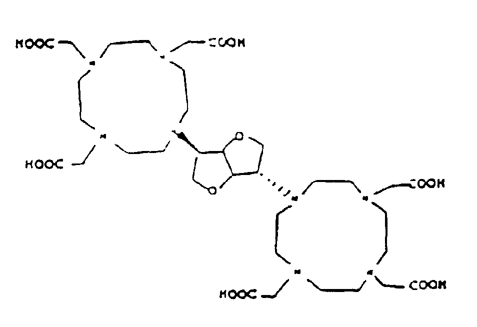

8. Ligand of formula

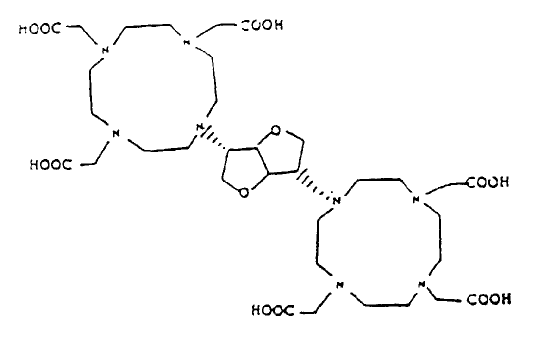

9. Ligand of formula

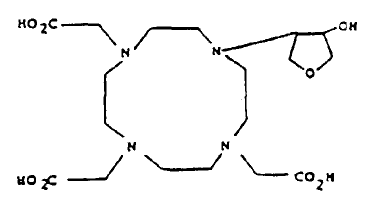

10. Process for the preparation of compounds of the general formula II according to claim 1, characterised by the following steps:
a) reaction of a compound of formula 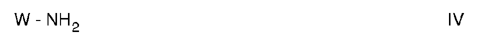 wherein W is as defined in claim 1 with a compound of formula 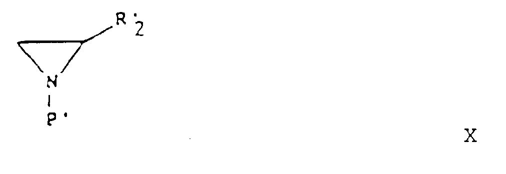 wherein R'₂ is as defined in claim 1 and P' represents a tosyl, mesyl, phenylsulphonyl, or 4-methoxyphenyl- sulphonyl group, to obtain a compound of formula XI 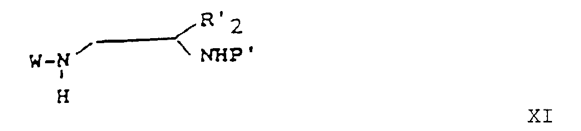
b) reaction of the compound of formula XI with a compound of formula XII 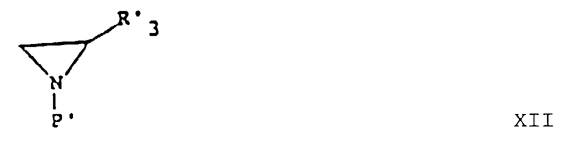 R'₃ being as defined in claim 1 and P' being as defined above to obtain the compound of formula XIII 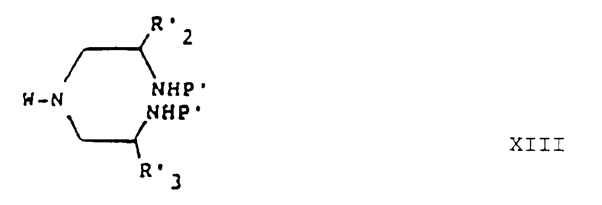
c) reaction of the compound of formula XIII with a compound of formula 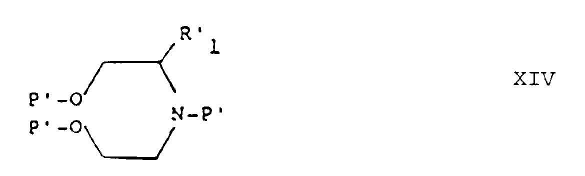 P' being as defined above and R'₁ being as defined in claim 1 to obtain a compound of formula XV 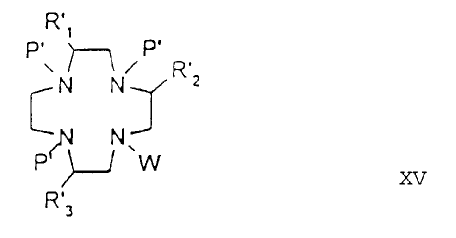
d) elimination of the group P', and
e) alkylation of the resulting compound to obtain a compound of formula II.

11. Neutral or ionic mono- or polymetallic complexes formed by at least one ligand of formula II according to claim 1, with at least one metallic ion selected from lanthanide ions, and ions of the transition metals, barium, bismuth, lead and the radioisotopes ^{99m}Tc, ¹¹¹In, ⁹⁰Y, ⁶⁴Cu and ¹⁶⁹Yb, and the salts of those complexes with pharmaceutically acceptable mineral or organic bases or with basic amino acids.

12. Mono- or bimetallic complexes according to claim 11, wherein the metallic ion is selected from gadolinium, europium, dysprosium, iron (Fe³⁺), manganese (Mn²⁺) and barium.

13. Diagnostic composition for magnetic resonance imagery, characterised in that it comprises at least one complex according to either claim 11 or claim 12.

## Claims (Claims for the following Contracting State(s) : ES)

1. Process for the preparation of compounds of the general formula II: 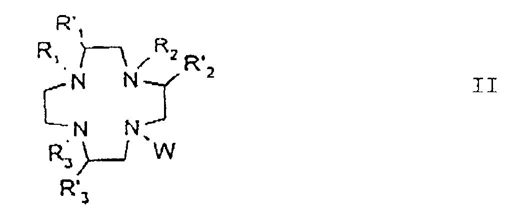 wherein R₁, R₂, R₃, which are the same or different, are selected from a hydrogen atom and the groups 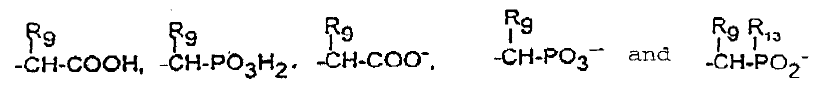 R₉ representing a hydrogen atom, a C₁-C₆ linear or branched chain alkyl group, a C₁-C₆ linear or branched chain hydroxyalkyl group, a C₁-C₆ linear or branched chain polyhydroxyalkyl group, or a C₁-C₆ linear or branched chain (C₁-C₆ alkoxy)alkyl group, and R₁₃ representing a C₁-C₆ linear or branched chain alkyl group, a C₁-C₆ linear or branched chain hydroxy- or polyhydroxyalkyl group, a C₁-C₆ linear or branched chain (C₁-C₆ alkoxy)alkyl group, or a C₁-C₆ linear or branched chain (C₁-C₆ hydroxy- or polyhydroxyalkoxy)alkyl group, with the proviso that at least two groups from among R₁, R₂ and R₃ represent 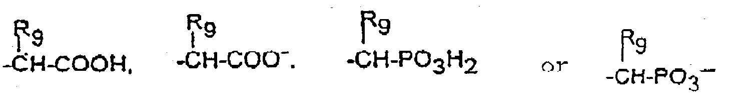 R'₁, R'₂ or R'₃, which are the same or different, representing a hydrogen atom, a C₁-C₆ linear or branched chain alkyl group, or a C₁-C₆ linear or branched chain hydroxyalkyl or polyhydroxyalkyl group, W is selected from the following groups: 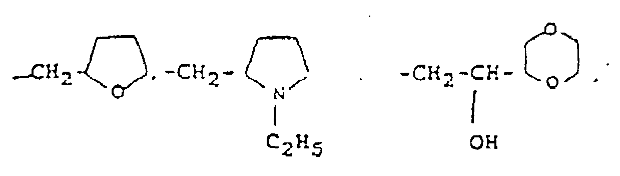 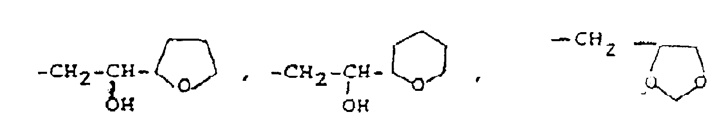 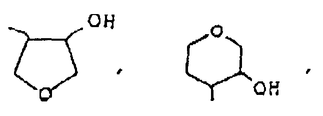 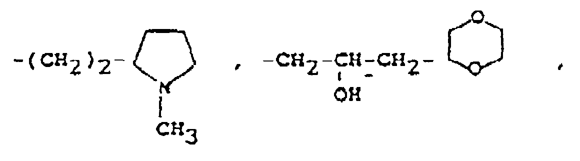 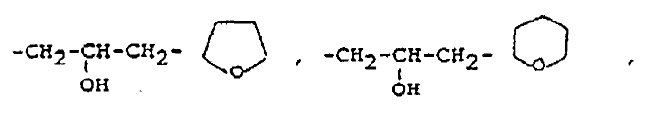 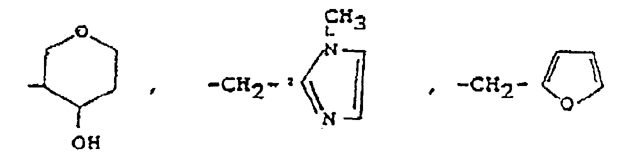 and 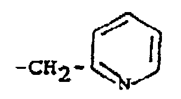 or W represents a group 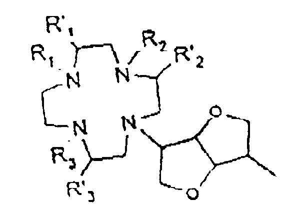 wherein R₁, R₂, R₃, R'₁, R'₂ and R'₃ are as defined above,
characterised by the following steps:
a) reaction of a compound of formula 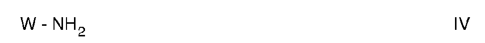 wherein W is as defined in claim 1 with a compound of formula 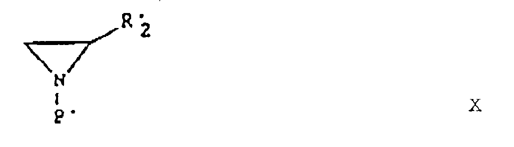 wherein R'₂ is as defined in claim 1 and P' represents a tosyl, mesyl, phenylsulphonyl, or 4-methoxyphenyl- sulponyl group, to obtain a compound of formula XI 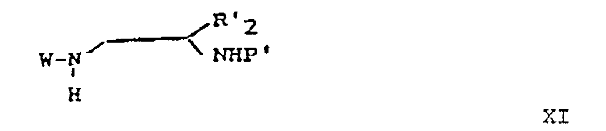
b) reaction of the compound of formula XI with a compound of formula XII 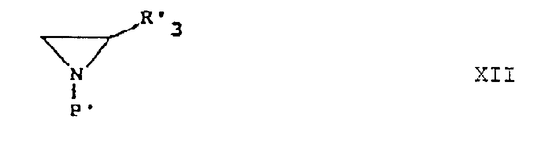 R'₃ being as defined in claim 1 and P' being as defincd above to obtain the compound of formula XIII 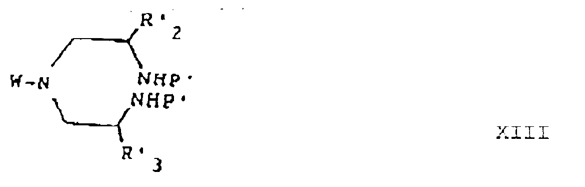
c) reaction of the compound of formula XIII with a compound of formula 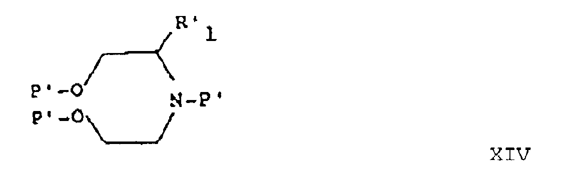 P' being as defined above and R'₁ being as defined in claim 1 to obtain a compound of formula XV 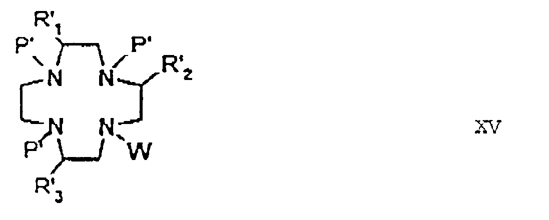
d) elimination of the group P', and
e) alkylation of the resulting compound to obtain a compound of formula II.

2. Process according to claim 1 for the preparation of a ligand of formula III 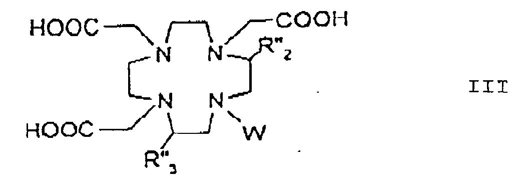 wherein R"₂ and R"₃ are selected from a hydrogen atom, the methyl group and the ethyl group and W represents the group

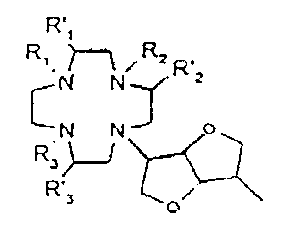

3. Process according to claim 1 for the preparation of a ligand of formula III 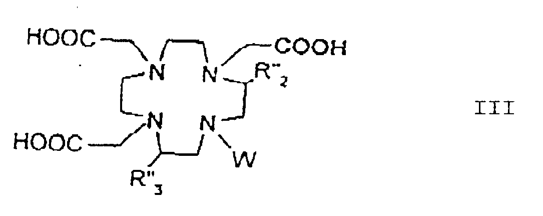 wherein R"₂ and R"₃ represent a hydrogen atom and W represents the groups: 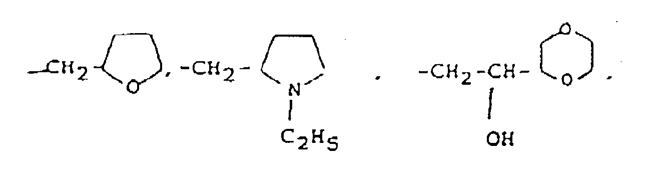 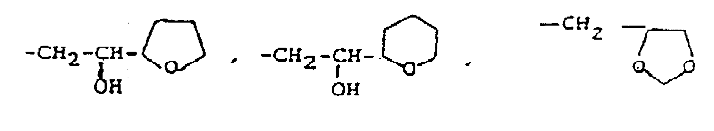 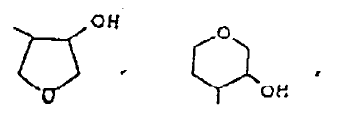 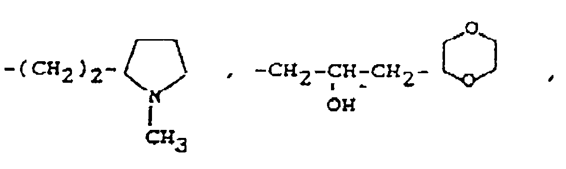 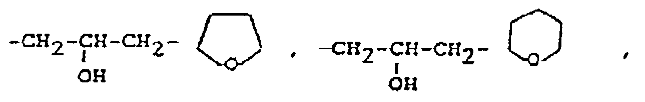 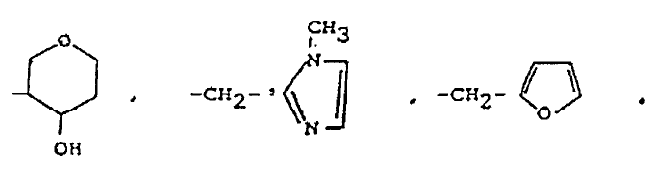 or

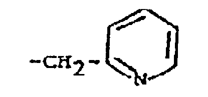

4. Process according to claim 1 for the preparation of compounds of formula

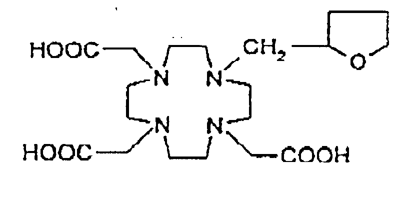

5. Process according to claim 1 for the preparation of compounds of formula III 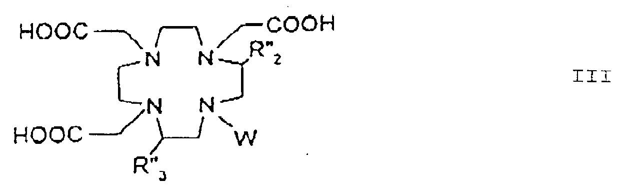 wherein R"₃ and R"₃ represent the methyl group and W represents

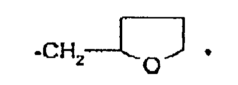

6. Process according to claim 1 for the preparation of a compound of formula

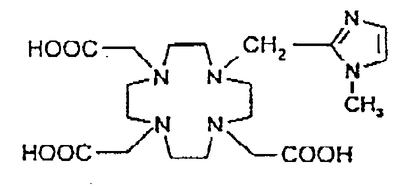

7. Process according to claim 1 for the preparation of compounds of formula

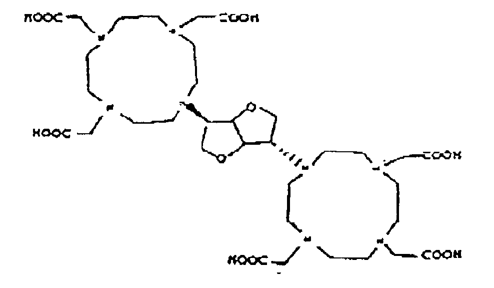

8. Process according to claim 1 for the preparation of compounds of formula

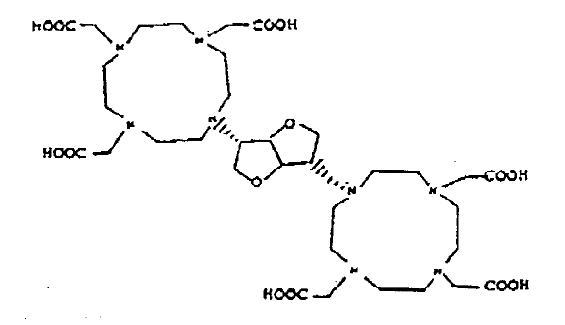

9. Process according to claim 1 for the preparation of compounds of formula

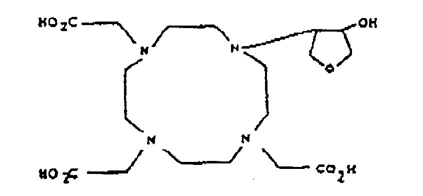

10. Process for the preparation of neutral or ionic mono- or polymetallic complexes formed by at least one ligand of formula II according to claim 1, with at least one metallic ion selected from lanthanide ions, and ions of the transition metals, barium, bismuth, lead and the radioisotopes ^{99m}Tc, ¹¹¹ln, ⁹⁰Y, ⁶⁴Cu and ¹⁶⁹Yb, and their salts with pharmaceutically acceptable mineral or organic bases or with basic amino acids, characterised in that a ligand Obtained by the process of any one of claims 1 to 9 is reacted with a salt or an oxide of the corresponding metal and, where appropriate, the resulting mixture is neutralised to obtain a salt.

11. Process according to claim 10 for the preparation of mono- or bimetallic complexes, wherein the metallic ion is selected from gadolinium, europium, dysprosium, iron (Fe³⁺), manganese (Mn²⁺) and barium.

12. Ligands of formula II: 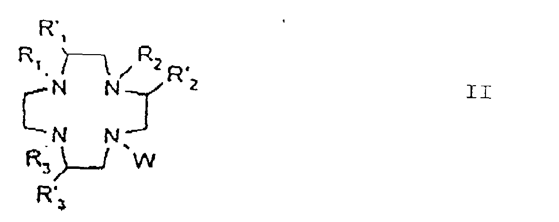 wherein R₁, R₂, R₃, which are the same or different, are selected from a hydrogen atom and the groups 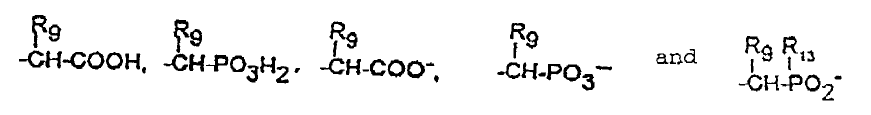 R₉ representing a hydrogen atom, a C₁-C₆ linear or branched chain alkyl group, a C₁-C₆ linear or branched chain hydroxyalkyl group, a C₁-C₆ linear or branched chain polyhydroxyalkyl group, or a C₁-C₆ linear or branched chain (C₁-C₆ alkoxy)alkyl group, and R₁₃ representing a C₁-C₆ linear or branched chain alkyl group, a C₁-C₆ linear or branched chain hydroxy- or polyhydroxyalkyl group, a C₁-C₆ linear or branched chain (C₁-C₆ alkoxy)alkyl group, or a C₁-C₆ linear or branched chain (C₁-C₆ hydroxy or polyhydroxyalkoxy)alkyl group, with the proviso that at least two groups from among R₁, R₂ and R₃ represent 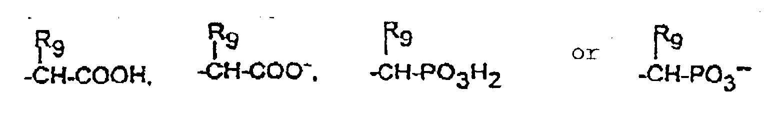 R'₁, R'₂ or R'₃, which are the same or different, represent ing a hydrogen atom, a C₁-C₆ linear or branched chain alkyl group, or a C₁-C₆ linear or branched chain hydroxyalkyl or polyhydroxyalkyl group, W is selected from the following groups: 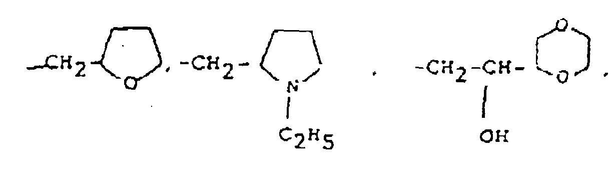 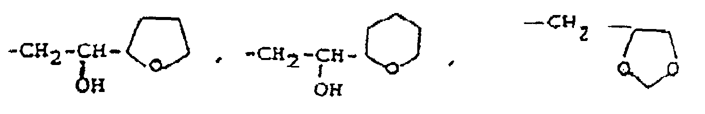 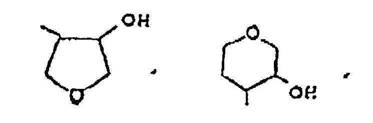 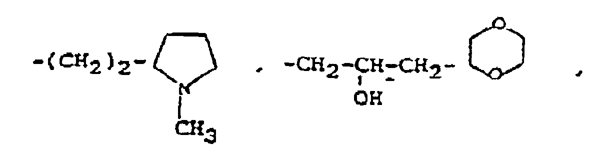 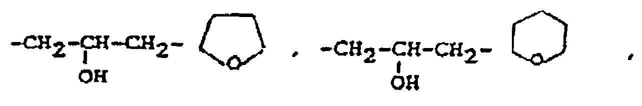 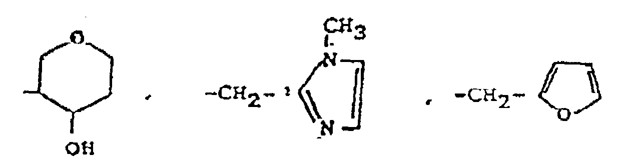 and 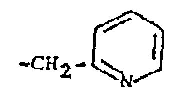 or W represents a group 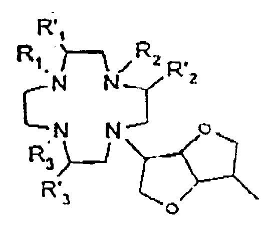 wherein R₁, R₂, R₃, R'₁, R'₂ and R'₃ are as defined above,
and the salts of those compounds formed with mineral or organic bases or with basic amino acids.
